# EUROPEAN PATENT SPECIFICATION

(11) **EP 1 756 316 B1**
(45) Date of publication and mention of the grant of the patent: **16.09.2009**
(21) Application number: 05764662.2
(22) Date of filing: 14.06.2005
(51) Int. Cl.: C12Q 1/68

(54) **HUMAN OBESITY SUSCEPTIBILITY GENE ENCODING A POTASSIUM VOLTAGE-GATED CHANNEL AND USES THEREOF**
FÜR EINEN SPANNUNGSGESTEUERTEN KALIUMKANAL KODIERENDES HUMANES ADIPOSITAS-SUSZEPTIBILITÄTSSGEN UND DESSEN VERWENDUNG
GENE HUMAIN DE SUSCEPTIBILITE A L'OBESITE CODANT POUR UN CANAL POTASSIQUE COMMANDE PAR TENSION ET UTILISATIONS DUDIT GENE

(30) Priority: 14.06.2004 US 578829 P
(43) Date of publication of application: 28.02.2007
(73) Proprietor: Integragen, 91000 EVRY (FR)
(72) Inventor: PHILIPPI, Anne, F-77310 St. Fargeau Ponthierry (FR); ROUSSEAU, Francis, F-91300 Savigny sur Orge (FR); ROSCHMANN, Elke, 89179 Beimerstetten (DE)
(74) Representative: Chajmowicz, Marion
(86) International application number: PCT/IB2005/002356
(87) International publication number: WO 2005/123949

(56) References cited:
- WO-A-01/66800
- WO-A-02/28999
- WO-A-20/04035755
- US-B1- 6 682 888

### SEQUENCE LISTING

<110> INTEGRAGEN
<120> HUMAN OBESITY SUSCEPTIBILITY GENE ENCODING A POTASSIUM VOLTAGE-GATED CHANNEL AND USES THEREOF
<130> B0292WO
<150> US 60/578,829
   <151> 2004-06-14
<160> 16
<170> PatentIn version 3.1
<210> 1
   <211> 3171
   <212> DNA
   <213> Homo sapiens
<220>
   <221> CDS
   <222> (179)..(1282)
   <223>
<400> 1
   gctgcaccaa cttcctgttg ggtgctgtgg tcccaaggcg cgtgctttgc agcagatgtg      60
   tgacctggcg ttcccctgag tgctcggaaa atggccttgt ctccggtcgt tcccgttcag     120
   gagcagaatt ttcccactgt aaaaaaccga gcaagtctgg ataagtgagg ctggctcc       178
   atg tat cca gaa tca acg acg ggc tcc ccg gct cgg ctc tcg ctg cgg       226
   Met Tyr Pro Glu Ser Thr Thr Gly Ser Pro Ala Arg Leu Ser Leu Arg
   1               5                   10                  15
   cag acg ggc tcc ccc ggg atg atc tac agt act cgg tat ggg agt ccc       274
   Gln Thr Gly Ser Pro Gly Met Ile Tyr Ser Thr Arg Tyr Gly Ser Pro
               20                  25                  30
   aaa aga cag ctc cag ttt tac agg aac ctg ggc aag tct ggc ctg cgg       322
   Lys Arg Gln Leu Gln Phe Tyr Arg Asn Leu Gly Lys Ser Gly Leu Arg
           35                  40                  45
   gtc tcc tgc ctg gga ctt gga aca tgg gtg acc ttc gga ggc cag atc       370
   Val Ser Cys Leu Gly Leu Gly Thr Trp Val Thr Phe Gly Gly Gln Ile
       50                  55                  60
   acc gat gag atg gca gag cag ctc atg acc ttg gcc tat gat aat ggc      418
   Thr Asp Glu Met Ala Glu Gln Leu Met Thr Leu Ala Tyr Asp Asn Gly
   65                  70                  75                  80
   atc aac ctc ttc gat aca gca gaa gtc tac gca gcc ggc aag gct gaa      466
   Ile Asn Leu Phe Asp Thr Ala Glu Val Tyr Ala Ala Gly Lys Ala Glu
                   85                  90                  95
   gtg gta ctg gga aac atc att aag aag aaa gga tgg agg cgg tcc agc      514
   Val Val Leu Gly Asn Ile Ile Lys Lys Lys Gly Trp Arg Arg Ser Ser
               100                 105                 110
   ctc gtc atc acc acc aag atc ttc tgg ggc gga aag gcg gag acg gag      562
   Leu Val Ile Thr Thr Lys Ile Phe Trp Gly Gly Lys Ala Glu Thr Glu
           115                 120                 125
   cgg ggc ctg tcc agg aag cac ata atc gaa ggt ctg aaa gct tcc ctg      610
   Arg Gly Leu Ser Arg Lys His Ile Ile Glu Gly Leu Lys Ala Ser Leu
       130                 135                 140
   gag cga ctg cag ctg gag tac gtg gat gtg gtg ttt gcc aac cgc ccg      658
   Glu Arg Leu Gln Leu Glu Tyr Val Asp Val Val Phe Ala Asn Arg Pro
   145                 150                 155                 160
   gac ccc aac acc ccg atg gaa gag acc gtc cgc gcc atg acc cac gtc      706
   Asp Pro Asn Thr Pro Met Glu Glu Thr Val Arg Ala Met Thr His Val
                   165                 170                 175
   atc aac cag ggg atg gcc atg tac tgg ggc acg tca cgc tgg agc tcc      754
   Ile Asn Gln Gly Met Ala Met Tyr Trp Gly Thr Ser Arg Trp Ser Ser
               180                 185                 190
   atg gag atc atg gag gcc tac tcc gtg gcc cgg cag ttc aac ctg acc      802
   Met Glu Ile Met Glu Ala Tyr Ser Val Ala Arg Gln Phe Asn Leu Thr
           195                 200                 205
   ccg ccc atc tgc gag cag gct gag tac cac atg ttc cag cgt gag aaa      850
   Pro Pro Ile Cys Glu Gln Ala Glu Tyr His Met Phe Gln Arg Glu Lys
       210                 215                 220
   gtg gag gtg cag ctg ccg gag ctg ttc cac aag ata gga gtg ggc gcc      898
   Val Glu Val Gln Leu Pro Glu Leu Phe His Lys Ile Gly Val Gly Ala
   225                 230                 235                 240
   atg acc tgg tcc cct ctg gcc tgt ggc att gtt tct ggc aag tac gac      946
   Met Thr Trp Ser Pro Leu Ala Cys Gly Ile Val Ser Gly Lys Tyr Asp
                   245                 250                 255
   agt ggc atc cca ccc tac tca aga gcc tcc ttg aag ggc tac cag tgg      994
   Ser Gly Ile Pro Pro Tyr Ser Arg Ala Ser Leu Lys Gly Tyr Gln Trp
               260                 265                 270
   ctg aag gac aag atc ctc agt gag gag ggc cgg cgc cag caa gcc aag     1042
   Leu Lys Asp Lys Ile Leu Ser Glu Glu Gly Arg Arg Gln Gln Ala Lys
           275                 280                 285
   ctg aag gag ctg cag gcc atc gcc gag cgc ctg ggc tgc acc ctg ccc     1090
   Leu Lys Glu Leu Gln Ala Ile Ala Glu Arg Leu Gly Cys Thr Leu Pro
       290                 295                 300
   cag ctg gcc ata gcc tgg tgc ctg agg aat gag gga gtc agc tcc gtg     1138
   Gln Leu Ala Ile Ala Trp Cys Leu Arg Asn Glu Gly Val Ser Ser Val
   305                 310                 315                 320
   ctc ctg ggg gcc tcc aat gcg gac cag ctc atg gag aac att ggg gca     1186
   Leu Leu Gly Ala Ser Asn Ala Asp Gln Leu Met Glu Asn Ile Gly Ala
                   325                 330                 335
   ata cag gtc ctt ccg aaa ctg tca tct tcc att atc cac gag att gat      1234
   Ile Gln Val Leu Pro Lys Leu Ser Ser Ser Ile Ile His Glu Ile Asp
               340                 345                 350
   agt att ttg ggc aat aaa ccc tac agc aaa aag gac tac aga tcc taa      1282
   Ser Ile Leu Gly Asn Lys Pro Tyr Ser Lys Lys Asp Tyr Arg Ser
           355                 360                 365
   gccgcccccg cccgcctgct cggacagttt ccgttccctc ctagtctctg ttcgctcgct  1342
   taagctgttt tgaagccaag tgaagagtgt ggtttgcatc caagagaaaa caccacactg  1402
   tgatgtcatc gggaaatgat ctcccaagtc gctgccagac accacccact gcttcgccgg  1462
   acaatgtcga agtccagtct gtgccgggga aggcactggt taggaaggat gttcaaacgg  1522
   tcccacccaa gcctgtcacc tctgctcatc ctccaagacc acccagcttt ctcccagcca  1582
   cagccaagat tcccaaagtc aaggcccaaa gatttccaag gttcccaaag tcaaggccag  1642
   gccaaggcct ggttgggtcc ttggggcggg cagggccagc ctctcctctg ctgagaatcc  1702
   ccacttggtg tagggggaga ggggaaaggg gtctggccca tcgaggggcc ccttctgcca  1762
   gggccttggt tgctggggca gggcctcccc actgggggtc ttcctccacc tcccactttc  1822
   caagggctcc aggaatctgg ggcctgacca cagattcctc tcccatcctt ttctgctcca  1882
   acctgcccca ctgggtcccg gcaggggcca tgcctaccaa gctcgagctg gcccttgacc  1942
   cccacccacc cccacccttg ctggcagggg cagggacccc agggggattg actctgcagt  2002
   ttgggagcca caaaaagcgt agcggtgtga tttctagctc agcctcccac cgtcttcctc  2062
   ctacacacca atgatgagcc tcatgccagt gaggcccgga gcgcttggga ggggtcccag  2122
   tggggcaggc ccctctgtct ggccacccct ctgtcctggc cccggaaggc cctgtggtca  2182
   tgtgctccta gctgcacggt ggctgctggc cacaccacgg caagtggcag caggggccgg  2242
   ccctgtgcac aaggatgcac tcctctcggc ccctgtagac tttctctaaa gccgcccgcc  2302
   agcccaggcc gctgctctgc accgagctgg tgggcttggg ttttgtggag cgcatgcttg  2362
   gaccctttca gtaaggaagg gtctttgggg ttttctgtgc ccatgacttg ggggctgcac  2422
   ccccacagca cccccacaat gtaggaaaag acctcaggga acctctccct ggaaagacgg  2482
   gcagggctgg ttagcccctc ccactgcctg acacctggga caggctgggc agaggggaga  2542
   gagggcagga caggccagag tgacgccccc gtgcagcttg ggccggaggg caagggatgc  2602
   cagtaagtct gcaggtgcgg ggtgccacct acaggcccag gcctgtgtcc caagcagtac  2662
   ccaggctttg cagaccacgc ggggcagggc tccactgaag ccacccccac ccctcgccag  2722
   ctagctccat agggaagcct gtgtctcctg cccccagggc gcaccctcag tgcaggcacc  2782
   tctgttcccg ctttgcccct ggaggagcca ctattccaga aggctccacc ctgccgtcct  2842
   gcgggagcct gctgtccagt cctggccggg ccaaggcctg ggaaactgtg aaagtcagaa  2902
   aggccagcgg ggagaggctg gggcgagggg aggaggggga tcagcttctg ctattaccga  2962
   ccccccttca tgctgcccct ggcgcctaga acccttgccc ctcctcatag accaagtccc  3022
   gggggtctcc actcagtcct gctgcctgct tcaccagaag cagccctgtg agtgtggggt  3082
   ggggaagtcc cttcccaacg gaggtcccag cctatggccc tgggcccagg tgggggtcgc    3142
   ctgcttcctt cccggacagg gtcctgcag                                      3171
<210> 2
   <211> 367
   <212> PRT
   <213> Homo sapiens
<400> 2
   Met Tyr Pro Glu Ser Thr Thr Gly Ser Pro Ala Arg Leu Ser Leu Arg
   1               5                   10                  15
   Gln Thr Gly Ser Pro Gly Met Ile Tyr Ser Thr Arg Tyr Gly Ser Pro
               20                  25                  30
   Lys Arg Gln Leu Gln Phe Tyr Arg Asn Leu Gly Lys Ser Gly Leu Arg
           35                  40                  45
   Val Ser Cys Leu Gly Leu Gly Thr Trp Val Thr Phe Gly Gly Gln Ile
       50                  55                  60
   Thr Asp Glu Met Ala Glu Gln Leu Met Thr Leu Ala Tyr Asp Asn Gly
   65                  70                  75                  80
   Ile Asn Leu Phe Asp Thr Ala Glu Val Tyr Ala Ala Gly Lys Ala Glu
                   85                  90                  95
   Val Val Leu Gly Asn Ile Ile Lys Lys Lys Gly Trp Arg Arg Ser Ser
               100                 105                 110
   Leu Val Ile Thr Thr Lys Ile Phe Trp Gly Gly Lys Ala Glu Thr Glu
           115                 120                 125
   Arg Gly Leu Ser Arg Lys His Ile Ile Glu Gly Leu Lys Ala Ser Leu
       130                 135                 140
   Glu Arg Leu Gln Leu Glu Tyr Val Asp Val Val Phe Ala Asn Arg Pro
   145                 150                 155                 160
   Asp Pro Asn Thr Pro Met Glu Glu Thr Val Arg Ala Met Thr His Val
                   165                 170                 175
   Ile Asn Gln Gly Met Ala Met Tyr Trp Gly Thr Ser Arg Trp Ser Ser
               180                 185                 190
   Met Glu Ile Met Glu Ala Tyr Ser Val Ala Arg Gln Phe Asn Leu Thr
           195                 200                 205
   Pro Pro Ile Cys Glu Gln Ala Glu Tyr His Met Phe Gln Arg Glu Lys
       210                 215                 220
   Val Glu Val Gln Leu Pro Glu Leu Phe His Lys Ile Gly Val Gly Ala
   225                 230                 235                 240
   Met Thr Trp Ser Pro Leu Ala Cys Gly Ile Val Ser Gly Lys Tyr Asp
                   245                 250                 255
   Ser Gly Ile Pro Pro Tyr Ser Arg Ala Ser Leu Lys Gly Tyr Gln Trp
               260                 265                 270
   Leu Lys Asp Lys Ile Leu Ser Glu Glu Gly Arg Arg Gln Gln Ala Lys
           275                 280                 285
   Leu Lys Glu Leu Gln Ala Ile Ala Glu Arg Leu Gly Cys Thr Leu Pro
       290                 295                 300
   Gln Leu Ala Ile Ala Trp Cys Leu Arg Asn Glu Gly Val Ser Ser Val
   305                 310                 315                 320
   Leu Leu Gly Ala Ser Asn Ala Asp Gln Leu Met Glu Asn Ile Gly Ala
                   325                 330                 335
   Ile Gln Val Leu Pro Lys Leu Ser Ser Ser Ile Ile His Glu Ile Asp
               340                 345                 350
   Ser Ile Leu Gly Asn Lys Pro Tyr Ser Lys Lys Asp Tyr Arg Ser
           355                 360                 365
<210> 3
   <211> 3129
   <212> DNA
   <213> Homo sapiens
<220>
   <221> CDS
   <222> (179)..(1240)
   <223>
<400> 3
   gctgcaccaa cttcctgttg ggtgctgtgg tcccaaggcg cgtgctttgc agcagatgtg 60
   tgacctggcg ttcccctgag tgctcggaaa atggccttgt ctccggtcgt tcccgttcag 120
   gagcagaatt ttcccactgt aaaaaaccga gcaagtctgg ataagtgagg ctggctcc 178
   atg tat cca gaa tca acg acg ggc tcc ccg gct cgg ctc tcg ctg cgg 226
   Met Tyr Pro Glu Ser Thr Thr Gly Ser Pro Ala Arg Leu Ser Leu Arg
   1               5                   10                  15
   cag acg ggc tcc ccc ggg atg atc tac agg aac ctg ggc aag tct ggc        274
   Gln Thr Gly Ser Pro Gly Met Ile Tyr Arg Asn Leu Gly Lys Ser Gly
               20                  25                  30
   ctg cgg gtc tcc tgc ctg gga ctt gga aca tgg gtg acc ttc gga ggc        322
   Leu Arg Val Ser Cys Leu Gly Leu Gly Thr Trp Val Thr Phe Gly Gly
           35                  40                  45
   cag atc acc gat gag atg gca gag cag ctc atg acc ttg gcc tat gat        370
   Gln Ile Thr Asp Glu Met Ala Glu Gln Leu Met Thr Leu Ala Tyr Asp
       50                  55                  60
   aat ggc atc aac ctc ttc gat aca gca gaa gtc tac gca gcc ggc aag        418
   Asn Gly Ile Asn Leu Phe Asp Thr Ala Glu Val Tyr Ala Ala Gly Lys
   65                  70                  75                  80
   gct gaa gtg gta ctg gga aac atc att aag aag aaa gga tgg agg cgg        466
   Ala Glu Val Val Leu Gly Asn Ile Ile Lys Lys Lys Gly Trp Arg Arg
                   85                  90                  95
   tcc agc ctc gtc atc acc acc aag atc ttc tgg ggc gga aag gcg gag        514
   Ser Ser Leu Val Ile Thr Thr Lys Ile Phe Trp Gly Gly Lys Ala Glu
               100                 105                 110
   acg gag cgg ggc ctg tcc agg aag cac ata atc gaa ggt ctg aaa gct        562
   Thr Glu Arg Gly Leu Ser Arg Lys His Ile Ile Glu Gly Leu Lys Ala
           115                 120                 125
   tcc ctg gag cga ctg cag ctg gag tac gtg gat gtg gtg ttt gcc aac        610
   Ser Leu Glu Arg Leu Gln Leu Glu Tyr Val Asp Val Val Phe Ala Asn
       130                 135                 140
   cgc ccg gac ccc aac acc ccg atg gaa gag acc gtc cgc gcc atg acc        658
   Arg Pro Asp Pro Asn Thr Pro Met Glu Glu Thr Val Arg Ala Met Thr
   145                 150                 155                 160
   cac gtc atc aac cag ggg atg gcc atg tac tgg ggc acg tca cgc tgg        706
   His Val Ile Asn Gln Gly Met Ala Met Tyr Trp Gly Thr Ser Arg Trp
                   165                 170                 175
   agc tcc atg gag atc atg gag gcc tac tcc gtg gcc cgg cag ttc aac        754
   Ser Ser Met Glu Ile Met Glu Ala Tyr Ser Val Ala Arg Gln Phe Asn
               180                 185                 190
   ctg acc ccg ccc atc tgc gag cag gct gag tac cac atg ttc cag cgt        802
   Leu Thr Pro Pro Ile Cys Glu Gln Ala Glu Tyr His Met Phe Gln Arg
           195                 200                 205
   gag aaa gtg gag gtg cag ctg ccg gag ctg ttc cac aag ata gga gtg        850
   Glu Lys Val Glu Val Gln Leu Pro Glu Leu Phe His Lys Ile Gly Val
       210                 215                 220
   ggc gcc atg acc tgg tcc cct ctg gcc tgt ggc att gtt tct ggc aag        898
   Gly Ala Met Thr Trp Ser Pro Leu Ala Cys Gly Ile Val Ser Gly Lys
   225                 230                 235                 240
   tac gac agt ggc atc cca ccc tac tca aga gcc tcc ttg aag ggc tac        946
   Tyr Asp Ser Gly Ile Pro Pro Tyr Ser Arg Ala Ser Leu Lys Gly Tyr
                   245                 250                 255
   cag tgg ctg aag gac aag atc ctc agt gag gag ggc cgg cgc cag caa        994
   Gln Trp Leu Lys Asp Lys Ile Leu Ser Glu Glu Gly Arg Arg Gln Gln
               260                 265                 270
   gcc aag ctg aag gag ctg cag gcc atc gcc gag cgc ctg ggc tgc acc       1042
   Ala Lys Leu Lys Glu Leu Gln Ala Ile Ala Glu Arg Leu Gly Cys Thr
           275                 280                 285
   ctg ccc cag ctg gcc ata gcc tgg tgc ctg agg aat gag gga gtc agc      1090
   Leu Pro Gln Leu Ala Ile Ala Trp Cys Leu Arg Asn Glu Gly Val Ser
       290                 295                 300
   tcc gtg ctc ctg ggg gcc tcc aat gcg gac cag ctc atg gag aac att      1138
   Ser Val Leu Leu Gly Ala Ser Asn Ala Asp Gln Leu Met Glu Asn Ile
   305                 310                 315                 320
   ggg gca ata cag gtc ctt ccg aaa ctg tca tct tcc att atc cac gag      1186
   Gly Ala Ile Gln Val Leu Pro Lys Leu Ser Ser Ser Ile Ile His Glu
                   325                 330                 335
   att gat agt att ttg ggc aat aaa ccc tac agc aaa aag gac tac aga      1234
   Ile Asp Ser Ile Leu Gly Asn Lys Pro Tyr Ser Lys Lys Asp Tyr Arg
               340                 345                 350
   tcc taa gccgcccccg cccgcctgct cggacagttt ccgttccctc ctagtctctg       1290
   Ser
   ttcgctcgct taagctgttt tgaagccaag tgaagagtgt ggtttgcatc caagagaaaa    1350
   caccacactg tgatgtcatc gggaaatgat ctcccaagtc gctgccagac accacccact    1410
   gcttcgccgg acaatgtcga agtccagtct gtgccgggga aggcactggt taggaaggat    1470
   gttcaaacgg tcccacccaa gcctgtcacc tctgctcatc ctccaagacc acccagcttt    1530
   ctcccagcca cagccaagat tcccaaagtc aaggcccaaa gatttccaag gttcccaaag    1590
   tcaaggccag gccaaggcct ggttgggtcc ttggggcggg cagggccagc ctctcctctg    1650
   ctgagaatcc ccacttggtg tagggggaga ggggaaaggg gtctggccca tcgaggggcc    1710
   ccttctgcca gggccttggt tgctggggca gggcctcccc actgggggtc ttcctccacc    1770
   tcccactttc caagggctcc aggaatctgg ggcctgacca cagattcctc tcccatcctt    1830
   ttctgctcca acctgcccca ctgggtcccg gcaggggcca tgcctaccaa gctcgagctg    1890
   gcccttgacc cccacccacc cccacccttg ctggcagggg cagggacccc agggggattg    1950
   actctgcagt ttgggagcca caaaaagcgt agcggtgtga tttctagctc agcctcccac    2010
   cgtcttcctc ctacacacca atgatgagcc tcatgccagt gaggcccgga gcgcttggga    2070
   ggggtcccag tggggcaggc ccctctgtct ggccacccct ctgtcctggc cccggaaggc    2130
   cctgtggtca tgtgctccta gctgcacggt ggctgctggc cacaccacgg caagtggcag    2190
   caggggccgg ccctgtgcac aaggatgcac tcctctcggc ccctgtagac tttctctaaa    2250
   gccgcccgcc agcccaggcc gctgctctgc accgagctgg tgggcttggg ttttgtggag    2310
   cgcatgcttg gaccctttca gtaaggaagg gtctttgggg ttttctgtgc ccatgacttg    2370
   ggggctgcac ccccacagca cccccacaat gtaggaaaag acctcaggga acctctccct    2430
   ggaaagacgg gcagggctgg ttagcccctc ccactgcctg acacctggga caggctgggc    2490
   agaggggaga gagggcagga caggccagag tgacgccccc gtgcagcttg ggccggaggg    2550
   caagggatgc cagtaagtct gcaggtgcgg ggtgccacct acaggcccag gcctgtgtcc    2610
   caagcagtac ccaggctttg cagaccacgc ggggcagggc tccactgaag ccacccccac    2670
   ccctcgccag ctagctccat agggaagcct gtgtctcctg cccccagggc gcaccctcag    2730
   tgcaggcacc tctgttcccg ctttgcccct ggaggagcca ctattccaga aggctccacc 2790
   ctgccgtcct gcgggagcct gctgtccagt cctggccggg ccaaggcctg ggaaactgtg 2850
   aaagtcagaa aggccagcgg ggagaggctg gggcgagggg aggaggggga tcagcttctg 2910
   ctattaccga ccccccttca tgctgcccct ggcgcctaga acccttgccc ctcctcatag 2970
   accaagtccc gggggtctcc actcagtcct gctgcctgct tcaccagaag cagccctgtg 3030
   agtgtggggt ggggaagtcc cttcccaacg gaggtcccag cctatggccc tgggcccagg 3090
   tgggggtcgc ctgcttcctt cccggacagg gtcctgcag 3129
<210> 4
   <211> 353
   <212> PRT
   <213> Homo sapiens
<400> 4
   Met Tyr Pro Glu Ser Thr Thr Gly Ser Pro Ala Arg Leu Ser Leu Arg
   1 5 10 15
   Gln Thr Gly Ser Pro Gly Met Ile Tyr Arg Asn Leu Gly Lys Ser Gly
   20 25 30
   Leu Arg Val Ser Cys Leu Gly Leu Gly Thr Trp Val Thr Phe Gly Gly
   35 40 45
   Gln Ile Thr Asp Glu Met Ala Glu Gln Leu Met Thr Leu Ala Tyr Asp
   50 55 60
   Asn Gly Ile Asn Leu Phe Asp Thr Ala Glu Val Tyr Ala Ala Gly Lys
   65 70 75 80
   Ala Glu Val Val Leu Gly Asn Ile Ile Lys Lys Lys Gly Trp Arg Arg
   85 90 95
   Ser Ser Leu Val Ile Thr Thr Lys Ile Phe Trp Gly Gly Lys Ala Glu
   100 105 110
   Thr Glu Arg Gly Leu Ser Arg Lys His Ile Ile Glu Gly Leu Lys Ala
   115 120 125
   Ser Leu Glu Arg Leu Gln Leu Glu Tyr Val Asp Val Val Phe Ala Asn
   130 135 140
   Arg Pro Asp Pro Asn Thr Pro Met Glu Glu Thr Val Arg Ala Met Thr
   145 150 155 160
   His Val Ile Asn Gln Gly Met Ala Met Tyr Trp Gly Thr Ser Arg Trp
   165 170 175
   Ser Ser Met Glu Ile Met Glu Ala Tyr Ser Val Ala Arg Gln Phe Asn
               180                 185                 190
   Leu Thr Pro Pro Ile Cys Glu Gln Ala Glu Tyr His Met Phe Gln Arg
           195                 200                 205
   Glu Lys Val Glu Val Gln Leu Pro Glu Leu Phe His Lys Ile Gly Val
       210                 215                 220
   Gly Ala Met Thr Trp Ser Pro Leu Ala Cys Gly Ile Val Ser Gly Lys
   225                 230                 235                 240
   Tyr Asp Ser Gly Ile Pro Pro Tyr Ser Arg Ala Ser Leu Lys Gly Tyr
                   245                 250                 255
   Gln Trp Leu Lys Asp Lys Ile Leu Ser Glu Glu Gly Arg Arg Gln Gln
               260                 265                 270
   Ala Lys Leu Lys Glu Leu Gln Ala Ile Ala Glu Arg Leu Gly Cys Thr
           275                 280                 285
   Leu Pro Gln Leu Ala Ile Ala Trp Cys Leu Arg Asn Glu Gly Val Ser
       290                 295                 300
   Ser Val Leu Leu Gly Ala Ser Asn Ala Asp Gln Leu Met Glu Asn Ile
   305                 310                 315                 320
   Gly Ala Ile Gln Val Leu Pro Lys Leu Ser Ser Ser Ile Ile His Glu
                   325                 330                 335
   Ile Asp Ser Ile Leu Gly Asn Lys Pro Tyr Ser Lys Lys Asp Tyr Arg
               340                 345                 350
   Ser
<210> 5
   <211> 822
   <212> DNA
   <213> homo sapiens
<220>
   <221> misc_feature
   <222> (272)..(272)
   <223> SNP2 s = g or c
<400> 5
   cctcacacag gtgtgccgtc tcttgctcca ggaatctctc cttggccagc cacctcttgg      60
   ccaggttaag gatcccccag ccctgcacag atctcagcat gttctcggca cttcatgtta     120
   taattatctg ctgtggtccc tctgcatgag tggcaagtct gaggtctcat tcagacttca     180
   aaacatgcac caagaagcac agtgcctaga acacaagggt tgagggaacc ttggccgaat     240
   gaaagaatgg aatctgccca ggctgcaaac asggcttcag gggagtggga gcccattggt     300
   ggaaaacccc agaagcgctg cttgatccca gctccttgga gtcaggccct acccttctgt     360
   tcactcttgg atccacctgg caggcccagg agacactgac cacatgctca gctccacggg     420
   ccacggtagg tgctcaataa aagcataatt ggggaatgaa tgcaggtggt tcctggaaga     480
   cagtgccatt tatcagctcg ttacagaccc aatcaccgtc gaggggaggg aggggcaggt     540
   ttgctccggg tttgcagctc ctcctgtcag aaaacctttg gaattaaggc attggcatta     600
   attgcgatag atcctctgaa aagccttcct gtttataatt tggacaaatt gccccacatc     660
   ctctctcgct gcgtgtctgt gtgtgatgtg cctcagggag gtattgattt atttttggtt     720
   ataagtgaga gttataccgt gttggtggct gctccctgta gatgtgggga catggccttc     780
   tcctggaggc agcagagata tttgacaccc agacccttca tc                        822
<210> 6
   <211> 51
   <212> DNA
   <213> Homo sapiens
<220>
   <221> misc_feature
   <222> (26)..(26)
   <223> SNP17 r = a or g
<400> 6
   tgtgtttctt ttcagaaatt tgaggraggg tgccaaaaca aagactagtg t 51
<210> 7
   <211> 401
   <212> DNA
   <213> Homo sapiens
<220>
   <221> misc_feature
   <222> (201)..(201)
   <223> SNP23 r = a or g
<400> 7
   tgttcagaat tcctggcaag gtgaacagtg acaggagctc tggtgttagt gaagccacag 60
   ttagacgggg tgggagggcg gcccctggtg ccaccagctg gccggggttt taagcccaag 120
   atggcttcag ttctgtctgc ggaggcagcg cttagggaat agtcgccgtg tgagaggagg 180
   gacttgcctg gtgtggaccc ragtcgcggc cacagtcatg tcagggttgg ttgtggcctc 240
   agatttggcc gccccggaac cacccatgac cttgggctcc cctggctgat tgggctgcag 300
   gcgcccagga gccccttgct tgcacatcct gagaggctgt gctctgaccc gcctctctcg 360
   ctcttgaaga cagaccccaa aggtgtcttc gtgctgccgc c 401
<210> 8
   <211> 121
   <212> DNA
   <213> Homo sapiens
<220>
   <221> misc_feature
   <222> (61)..(61)
   <223> SNP33 y = t or c
<400> 8
   aagagaggag ggaccctcgc cccagcgaca cagtggattc ttcccaatat tcaaacatgg 60
   ytttgagaca cagcaatctg ctggttatag ctccctctca gcctcatggg gatattgtga 120
   t 121
<210> 9
   <211> 401
   <212> DNA
   <213> Homo sapiens
<220>
   <221> misc_feature
   <222> (201)..(201)
   <223> SNP34 y = t or c
<400> 9
   tgacatcaca gtgtggtgtt ttctcttgga tgcaaaccac actcttcact tggcttcaaa 60
   acagcttaag cgagcgaaca gagactagga gggaacggaa actgtccgag caggcgggcg 120
   ggggcggctt aggatctgta gtcctttttg ctgtagggtt tattgcccaa aatactatca 180
   atctcgtgga taatggaaga ygacagtttc ggaaggacct gcgtgcaaga acaacaacaa 240
   atcagaatcc caccaacaag acgggcctgg cctggggagg aggtggggcc aggctcttcc 300
   agatggtcgg ctggccaggc ccgggttgcc ccatcacatc tgggctccat ctgaggcaga 360
   tggaggccac gctgcttttg tgtttcagaa ggtttggggc a 401
<210> 10
   <211> 401
   <212> DNA
   <213> Homo sapiens
<220>
   <221> misc_feature
   <222> (201)..(201)
   <223> SNP38 y = c or t
<400> 10
   tgacatcaca gtgtggtgtt ttctcttgga tgcaaaccac actcttcact tggcttcaaa      60
   acagcttaag cgagcgaaca gagactagga gggaacggaa actgtccgag caggcgggcg     120
   ggggcggctt aggatctgta gtcctttttg ctgtagggtt tattgcccaa aatactatca      180
   atctcgtgga taatggaaga ygacagtttc ggaaggacct gcgtgcaaga acaacaacaa     240
   atcagaatcc caccaacaag acgggcctgg cctggggagg aggtggggcc aggctcttcc     300
   agatggtcgg ctggccaggc ccgggttgcc ccatcacatc tgggctccat ctgaggcaga     360
   tggaggccac gctgcttttg tgtttcagaa ggtttggggc a                         401
<210> 11
   <211> 514
   <212> DNA
   <213> Homo sapiens
<220>
   <221> misc_feature
   <222> (482)..(482)
   <223> SNP42 y = c or t
<400> 11
   gctgggccct gctctccgca cccacccaca gccttggcta gtgcccccag gacaggcctg      60
   tgtgagaaag tgaccctctg cccgctgcag ctggtgcggg gaaggccctt cctactaagt     120
   ggaaaacaaa gggaagaggc tgatagaatc attttgtgac ttataattgg ctacctcata     180
   actcagacaa aaaaagccca aaaacctttc atagagtaag ttcaattttt aaaaacaaag     240
   gtattttaaa atgaacactt gagaagccaa atgaggtttc ttgtgtgctc tggggatccc     300
   ccggccactg tgtcacgccg tgccggccgc tccacaggca actgtccctg agcctcccac     360
   ggaaccttgg gccctgcctg acctcagagc cagaagccag ttggcctaag tgggccctgg     420
   tttgcataat agactctcac ccagggtgga ggggtgacac tggggatggg cagctatggg     480
   tygctggggg acatctgtgg tcatttgctc taga                                 514
<210> 12
   <211> 701
   <212> DNA
   <213> Homo sapiens
<220>
   <221> misc_feature
   <222> (201)..(201)
   <223> SNP47 r = g or a
<400> 12
   cacagacagc cgtgtgccca ggctcaccca cccgaccgcc tcagctcatg catgagacct    60
   gttcacgtgc tgtgacccag ccgctgtgtg cagccccccg gcgcctctct gccggccccg    120
   gggagagctg gggcaggcac acacgggtga acaggtctca gttctggtgc tcaggctgag    180
   ggtgtgcatg gtgcatctgg rtgccaccac ttgggatctc aaaggcctcg ccacacctca    240
   ccaggataaa ggggattcca agaatggcct tgactcagag agtgggcatg gatattccag    300
   gagcctagtt ttaaagagct ttgaggctgg acatggtggc ttatacccat aatcccaaca    360
   ctttgggaag ccaaggaggc agaaggatca ttttagtcca gatgctcaag accagcctgg    420
   gcaacataga gagactccat ctctacaaaa acattaaaaa accagcaggg cctggtggtg    480
   tgcacctgca gtcccagctg cttgggaggc tgaggcagga gtgctgcttc aggtcagatg    540
   cagtgagctg tgatctcgcc actgcactcc agcctgggta acagagcaag acaaccctgt    600
   cttttttttt tttttttttc agatggagtt ttgctcttgt cacccaggat ggagtgcaat    660
   ggtatgatct cggctcactg aacctctgcc tcccgggttc a                        701
<210> 13
   <211> 668
   <212> DNA
   <213> Homo sapiens
<220>
   <221> misc_feature
   <222> (81)..(81)
   <223> SNP49 r = g or a
<400> 13
   tgctgccagt ggaatgctgg gataacccca gtgtcggggg tcctggggca gcctgtgtac     60
   tgctggatgc ttgctttcaa rtactctgct ggggtaagac tctggggcag tttccatccc    120
   ttccttgggc ttcagcttgc tggactggcc ctgagaactg ggaggggatg gcttcatgaa    180
   gtcattttga tgggggatga agatagttgc tgtagaggaa gaggatgaga gagctgcttg    240
   gcctctggaa ggtgggagga gctgtcgcag gctgcacagg ggctctgagg tcgagttcgt    300
   ttagttcatt gattaagcag tctaccaggt ggccagcaag gctttgtggg tgcttgggat    360
   gcatttaagg gaactaaaca aaggtgcttg tgcagccgac ctgcttgcca ggcaatgcag    420
   ataacagata agtccatctg cctgcagtag acggtgagga agtgctgccg aagaagaaaa    480
   agcagaggcg ggccaagggg ttggggagtt gtgagtgggg ttcgattgcg attttaagta    540
   gccaggtctc tagagtctta ctgagaagtg agatataagc aaagactcaa agaggtgaga    600
   agttgaccaa gtgggtgact ggggagcatc tgggcagaaa gagaaccagg gcgctgggta    660
   ggaggatc                                                             668
<210> 14
   <211> 51
   <212> DNA
   <213> Homo sapiens
<220>
   <221> misc_feature
   <222> (26)..(26)
   <223> SNP51 m = a or c
<400> 14
   actctcaaag acactgggcc tggaamcact gcctaggggc cgaaaggaag g 51
<210> 15
   <211> 401
   <212> DNA
   <213> Homo sapiens
<220>
   <221> misc_feature
   <222> (201)..(201)
   <223> SNP53 s = g or c
<400> 15
   acgggacccg gggcgccacg taaaggggtg ctccgggccc caattcaggc ccgaaacccc 60
   aactcctagc cttgggcctg tgcaagtctt accccattcg ccaatgctgg tcgcctttca 120
   cctccttgct ttgagcaccc aaccaggacg catagccact cacaggcttg gacaactgga 180
   aaccaacggc caacccctca stgtttttag ggctttgaat tgtgaatagg tctggactcc 240
   tctgacgtca ctggaggtgg tactagttgg ccactggtgg tgtcagttca gaggccagac 300
   agctcactgc ccttaagttt ggtcacaggg aacccccaaa cgtgaagagg caaaaagggc 360
   aactgaaggg gagggagatt ccagttcagg ccttgcagaa a 401
<210> 16
   <211> 572
   <212> DNA
   <213> Homo sapiens
<220>
   <221> misc_feature
   <222> (211)..(211)
   <223> SNP69 y = t or c
<400> 16
   cacccacatg acctcagaag agctccaggc tccacgacct gtccagaggc agcaaggtgg 60
   atgcagagcc cgggggtccc ctgggcttcc tggattgaga ggcagtcccc ctccaccctt 120
   ttagcactga ctcagctggc gattcctctc atggttccag gattgccctg atgagccatc 180
   atgggggcac agcagcagga ttccagtcct ytccctgcta aatggtttcc tgtgtgggaa 240
   ttgaggctca ggttgtgcct ggaggagtga gactgggact gaagcagggc tagaggaccc 300
   aggggtctgg gatgggaaga aaccaccccc atcatcatgg cagggtgccc atggtggccc 360
   ttgaagagtg gtccccacat cttaaaatcc tacaacactt agaatcctag aacatagggc 420
   tggagccatg ttagagagca agtaatttga ccctaatatt ggaaataagg ggagaatagg 480
   gaagaaggaa atagaacaga agaccttgtc taagagaccc aggaggtcat tggagagctg 540
   tgaccagaaa ctcaggtagc tgaagatgga gc 572

## Description

### FIELD OF THE INVENTION

The present invention relates generally to the fields of genetics and medicine. The present invention more particularly discloses the identification of a human obesity susceptibility gene, which can be used for the diagnosis, prevention and treatment of obesity and related disorders, as well as for the screening of therapeutically active drugs. The invention more specifically discloses certain alleles of the potassium voltage-gated channel, shaker-related family, beta member 2 (KCNAB2) gene on chromosome 1 related to susceptibility to obesity and representing novel targets for therapeutic intervention. The present invention relates to particular mutations in the KCNAB2 gene and expression products, as well as to diagnostic tools and kits based on these mutations. The invention can be used in the diagnosis of predisposition to, detection, prevention and/or treatment of coronary heart disease and metabolic disorders, including hypoalphalipoproteinemia, familial combined hyperlipidemia, insulin resistant syndrome X or multiple metabolic disorder, coronary artery disease, diabetes and dyslipidemia.

### BACKGROUND OF THE INVENTION

Approximately three to eight percent of the total health costs of modem industrialized countries are currently due to the direct costs of obesity (Wolf, 1996). In Germany, the total costs (both direct and indirect) related to obesity and comorbid disorders were estimated at 21 billion German marks (29.4 US Dollar) in 1995 (Schneider, 1996). By 2030 these costs will rise by 50% even if the prevalence of obesity does not increase further.

Obesity is often defined simply as a condition of abnormal or excessive fat accumulation in adipose tissue, to the extent that health may be impaired. The underlying disease is the process of undesirable positive energy balance and weight gain. An abdominal fat distribution is associated with higher health risks than a gynoid fat distribution.

The body mass index (BMI; kg/m²) provides the most useful, albeit crude, population-level measure of obesity. It can be used to estimate the prevalence of obesity within a population and the risks associated with it. However, BMI does not account for body compositon or body fat distribution (WHO, 1998).

**Table 1: Classification of overweight in adults according to BMI (WHO, 1998)**

| Classification | BMI (kg/m²) | Risk of co-morbidities |
|---|---|---|
| Underweight | < 18.5 | Low (but risks of other clinical problems increased) |
| Normal range | 18.5 - 24.9 | Average |
| Overweight | ≥ 25 | |
| Pre-obese | 25 - 29.9 | Increased |
| Obese class I | 30 - 34.9 | Moderate |
| Obese class II | 35 - 39.9 | Severe |
| Obese class III | ≥ 40 | Very severe |

Obesity has also been defined using the 85^{th} and 95^{th} BMI-percentiles as cutoffs for definition of obesity and severe obesity. BMI-percentiles have been calculated within several populations; centiles for the German population based on the German National Nutrition Survey have been available since 1994 (Hebebrand et al., 1994, 1996). Because the WHO classification of the different weight classes can only be applied to adults, it has become customary to refer to BMI-percentiles for the definition of obesity in children and adolescents.

The recent rise in the prevalence of obesity is an issue of major concern for the health systems of several countries. According to reports of the Center of Disease Control and Prevention (CDC) there has been a dramatic increase in obesity in the United States during the past 20 years. In 1985 only a few states were participating in CDC's Behavioral Risk Factor Surveillance System (BRFSS) and providing obesity data. In 1991, four states were reporting obesity prevalence rates of 15-19 percent and no states reported rates at or above 20 percent. In 2002, 20 states have obesity prevalence rates of 15-19 percent; 29 states have rates of 20-24 percent; and one state reports a rate over 25 percent. Similar trends have been observed in other countries in Europe and South America.

Children and adolescents have not been exempt from this trend. Quite to the contrary, the increase in the USA has been substantial. Thus, between the 1960ies and 1990, overweight and obesity increased dramatically in 6 through to 17 year olds. The increments translate into relative increases of 40% using the 85^{th} BMI-centile (calculated in the 1960ies) as a cutoff and 100% upon use of the 95^{th} centile. In a cross sectional study of German children and adolescents treated as inpatients for extreme obesity between 1985 and 1995, a significant increase of the mean BMI of almost 2 kg/m² over this ten year period has been reported. Within this extreme group, the increments were most pronounced in the uppermost BMI ranges.

The mechanisms underlying this increase in the prevalence of obesity are unknown. Environmental factors have commonly been invoked as the underlying cause. Basically, both an increased caloric intake and a reduced level of physical activity have been discussed. In England the increase in obesity rates has been attributed to the latter mechanism. Thus, in this country, the average caloric intake even decreased somewhat within the last two decades, whereas indirect evidence stemming from the increases in hours spent watching television and from the average number of cars per household points to reduced levels of physical activity as the relevant causative factor.

Genetic factors have previously not been considered as a contributing cause. Quite to the contrary, the fact that the increased rates of obesity have been observed within the last two decades has been viewed as evidence that genetic factors cannot be held responsible. However, it has been proposed that an increase in the rate of assortative mating could very well constitute a genetic contribution to the observed phenomenon. This hypothesis is based on evidence suggesting that stigmatisation of obese individuals represents a rather recent social phenomenon, thus invariably having led to increased rates of assortative mating. As a consequence, the offspring have a higher loading with both additive and non-additive genetic factors underlying obesity. Indeed, an exceedingly high rate of (deduced) assortative mating amongst the parents of extremely obese children and adolescents has been observed.

Potentially life-threatening, chronic health problems associated with obesity fall into four main areas: 1) cardiovascular problems, including hypertension, chronic heart disease and stroke, 2) conditions associated with insulin resistance, namely Non-Insulin Dependent Diabetes Mellitus (NIDDM), 3) certain types of cancers, mainly the hormonally related and large-bowel cancers, and 4) gallbladder disease. Other problems associated with obesity include respiratory difficulties, chronic musculo-skeletal problems, skin problems and infertility (WHO, 1998).

The main currently available strategies for treating these disorders include dietary restriction, increments in physical activity, pharmacological and surgical approaches. In adults, long term weight loss is exceptional using conservative interventions. Present pharmacological interventions typically induce a weight loss of between five and fifteen kilograms; if the medication is discontinued, renewed weight gain ensues. Surgical treatments are comparatively successful and are reserved for patients with extreme obesity and/or with serious medical complications.

Recently, a 10 year old massively obese girl, in whom a leptin deficiency mutation had been detected, was treated successfully with recombinant leptin. This is the first individual who therapeutically profited from the detection of the mutation underlying her morbid obesity.

Several twin studies have been performed to estimate heritability of the BMI, some of which have encompassed over 1000 twin pairs. The results have been very consistent: The intrapair correlations among monozygotic twins were typically between 0.6 and 0.8, independent of age and gender. In one study, the correlations for monozygotic and dizygotic twins were basically the same, independent of whether the twins had been reared apart or together. Heritability of the BMI was estimated at 0.7; non-shared environmental factors explained the remaining 30% of the variance. Surprisingly, shared environmental factors did not explain a substantial proportion of the variance. Both hypercaloric and hypocaloric alimentation lead to similar degrees of weight gain or loss among both members of monozygotic twin pairs, indicating that genetic factors regulate the effect of environmentally induced variation of energy availability on body weight. Metabolic reactions and changes in body fat distribution upon overeating and undereating are also under genetic control (reviewed in Hebebrand et al., 1998).

A large adoption study has revealed that the BMI of adoptees is correlated with that of their biological parents and not with the BMI of the adoptive parents. Depending on the family study, the correlation between the BMI of sibs is between 0.2 and 0.4. Parent-offspring correlations are typically slightly lower. Segregation analyses have repeatedly suggested a major recessive gene effect. Based on these analyses, sample size calculations have been performed based on both concordant and discordant approaches. In contrast to the expectations, the concordant sib-pair approach was superior; a lower number of families were required to achieve the same power.

Family studies based on extremely obese young index patients, either mother or father or both, have a BMI > 90^{th} decile in the vast majority of the families. Based on index patients with a BMI > 95^{th} centile, approximately 20% of the respective families have a sib with a BMI > 90^{th} centile.

In conclusion, it is apparent that environmental factors interact with specific genotypes rendering an individual more or less susceptible to the development of obesity. Furthermore, despite the fact that major genes have been detected, it is necessary to consider that the spectrum reaches from such major genes to genes with an only minor influence.

The discovery of the leptin gene at the end of 1994 (Zhang et al., 1994) has been followed by a virtual explosion of scientific efforts to uncover the regulatory systems underlying appetite and weight regulation. It is currently the fastest growing biomedical field. This upswing has also resulted in large scaled molecular genetic activities which, due to obvious clinical interest, are basically all related to obesity in humans, rodents and other mammals (Hebebrand et al., 1998).

In this respect, many genes in which mutations lead to the presently known monogenic forms of obesity have been cloned in rodents. Systemic consequences of these mutations are currently being analysed. These models have provided insights into the complex regulatory systems involved in body weight regulation, the best known of which includes leptin and its receptor.

In mice, but also in pigs, over 15 quantitative trait loci (QTL) have been identified that are most likely relevant in weight regulation (Chagnon et al., 2003).

In humans, four exceedingly rare autosomal recessive forms of obesity have been described as of 1997. Mutations in the genes encoding for leptin, leptin receptor, prohormone convertase 1 and pro- opiomelanocortin (POMC) have been shown to cause massive obesity of an early onset type, associated with hyperphagia. Distinct additional clinical (e.g. red hair, primary amenorrhea) and/or endocrinological abnormalities (e.g. markedly altered serum leptin levels, lack of ACTH secretion) pinpointed to the respective candidate genes. Both the monogenic animal models and the human monogenic forms have led to new insights into the complex system underlying body weight regulation.

Very recently, the first autosomal dominant form of obesity was described in humans. Two different mutations within the melanocortin-4 receptor gene (*MC4R*) were observed to lead to extreme obesity in probands heterozygous for these variants. In contrast to the aforementioned findings, these mutations do not implicate readily obvious phenotypic abnormalities other than extreme obesity (Vaisse et al., 1998; Yeo et al., 1998). Interestingly, both groups detected the mutations by systematic screens in relatively small study groups (n=63 and n=43).

Hinney et al. (1999) screened the *MC4R* in a total of 492 obese children and adolescents. All in all, four individuals with two different mutations leading to haplo-insuffciency were detected. One was identical to that previously observed by Yeo et al. (1998). The other mutation, which was detected in three individuals, induced a stop mutation in the extracellular domain of the receptor. Approximately one percent of extremely obese individuals harbour haplo-insufficiency mutations in the *MC4R*. In addition to the two forms of haplo-insufficiency, Hinney et al. (1999) also detected additional mutations leading to both conservative and non-conservative amino acid exchanges. Interestingly, these mutations were mainly observed in the obese study group. The functional implications of these mutations are currently unknown.

The identification of individuals with *MC4R* mutations is interesting in the light of possible pharmacological interventions. Thus, intranasal application of adrenocorticotropn₄₋₁₀ (ACTH₄₋₁₀), representing a core sequence of all melanocortins, resulted in reduced weight, body fat mass and plasma leptin concentrations in healthy controls. The question arises as to how mutation carriers would react to this treatment, which could theoretically counterbalance their reduced receptor density.

The involvement of specific genes in weight regulation is further substantiated by data obtained from transgenic mice. For example, MC4R deficient mice develop early onset obesity (Huszar et al., 1997).

Different groups are conducting genome scans related to obesity or dependent phenotypes (BMI, leptin levels, fat mass, etc.). This approach appears very promising, because it is both systematic and model free. In addition, it has already been shown to be exceptionally successful. Thus, positive linkage results have been obtained even by analysing comparatively small study groups. More important, some findings have already been replicated. Each of the following regions has been identified by at least two independent groups: chromosome 1p32, chromosome 2p21, chromosome 6p21, chromosome 10 and chromosome 20q13 (Chagnon et al., 2003).

### SUMMARY OF THE INVENTION

The present invention now discloses the identification of the KCNAB2 gene, as human obesity susceptibility gene, which can be used for the diagnosis, prevention and treatment of obesity and related disorders, as well as for the screening of therapeutically active drugs.

The gene can be used in the diagnosis of predisposition to or protection from, detection, prevention and/or treatment of obesity, coronary heart disease and metabolic disorders, including hypoalphalipoproteinemia, familial combined hyperlipidemia, insulin resistant syndrome X or multiple metabolic disorder, coronary artery disease, diabetes and dyslipidemia, the method comprising detecting in a sample from the subject the presence of an alteration in the KCNAB2 gene or polypeptide, the presence of said alteration being indicative of the presence or predisposition to obesity or associated disorders. The presence of said alteration can also be indicative for protecting from obesity.

The description discloses a method of detecting the presence of or predisposition to obesity or an associated disorder in a subject, the method comprising detecting the presence of an alteration in the KCNAB2 gene locus in a sample from the subject, the presence of said alteration being indicative of the presence of or the predisposition to obesity or an associated disorder.

As well, a method of detecting the protection from obesity or an associated disorder in a subject, the method comprising detecting the presence of an alteration in the KCNAB2 gene locus in a sample from the subject, the presence of said alteration being indicative of the protection from obesity or an associated disorder.

As well, a method of assessing the response of a subject to a treatment of obesity or an associated disorder, the method comprising detecting the presence of an alteration in the KCNAB2 gene locus in a sample from the subject, the presence of said alteration being indicative of a particular response to said treatment.

As well a method of assessing the adverse effect in a subject to a treatment of obesity or an associated disorder, the method comprising detecting the presence of an alteration in the KCNAB2 gene locus in a sample from the subject, the presence of said alteration being indicative of an adverse effect to said treatment.

This description also relates to a method for preventing obesity or an associated disorder in a subject, comprising detecting the presence of an alteration in the KCNAB2 gene locus in a sample from the subject, the presence of said alteration being indicative of the predisposition to obesity or an associated disorder; and, administering a prophylactic treatment against obesity or an associated disorder.

In a preferred embodiment, said alteration is one or several SNP(s) or a haplotype of SNPs associated with obesity. More preferably, said haplotype associated with obesity comprises or consists of several SNPs selected in the group consisting of SNP2, SNP17, SNP23, SNP33, SNP34, SNP38, SNP42, SNP47, SNP49, SNP51, SNP53 and SNP69. Still more preferably, said haplotype is selected from the haplotypes disclosed in Table 5. More preferably, said SNP associated with obesity can be SNP33 or SNP34.

Preferably, the alteration in the KCNAB2 gene locus is determined by performing a hydridization assay, a sequencing assay, a microsequencing assay, or an allele-specific amplification assay.

Are disclosed as well, compositions of matter comprising primers, probes, and/or oligonucleotides, which are designed to specifically detect at least one SNP or haplotype associated with obesity in the genomic region including the KCNAB2 gene, or a combination thereof. More preferably, said haplotype associated with obesity comprises or consists of several SNPs selected in the group consisting of SNP2, SNP 17, SNP23, SNP33, SNP34, SNP38, SNP42, SNP47, SNP49, SNP51, SNP53 and SNP69. Still more preferably, said haplotype is selected from the haplotypes disclosed in Table 5. More preferably, said SNP associated with obesity can be SNP33 or SNP34.

As well methods of treating obesity and/or associated disorders in a subject through a modulation of KCNAB2 expression or activity. Such treatments use, for instance, KCNAB2 polypeptides, KCNAB2 DNA sequences (including antisense sequences and RNAi directed at the KCNAB2 gene locus), anti- KCNAB2 antibodies or drugs that modulate KCNAB2 expression or activity.

As well to methods of treating individuals who carry deleterious alleles of the KCNAB2 gene, including pre-symptomatic treatment or combined therapy, such as through gene therapy, protein replacement therapy or through the administration of KCNAB2 protein mimetics and/or inhibitors.

Is disclosed as well, the screening of drugs for therapy of obesity or associated disorder, based on the modulation of or binding to an allele of KCNAB2 gene associated with obesity or associated disorder or gene product thereof.

The description includes antibodies specific of KCNAB2 polypeptide fragments and derivatives of such antibodies, hybridomas secreting such antibodies, and diagnostic kits comprising those antibodies. More preferably, said antibodies are specific to a KCNAB2 polypeptide or a fragment thereof comprising an alteration, said alteration modifying the activity of KCNAB2.

The description also concerns a KCNAB2 gene or a fragment thereof comprising an alteration, said alteration modifying the activity of KCNAB2. The invention further concerns a KCNAB2 polypeptide or a fragment thereof comprising an alteration, said alteration modifying the activity of KCNAB2.

### LEGEND TO THE FIGURES

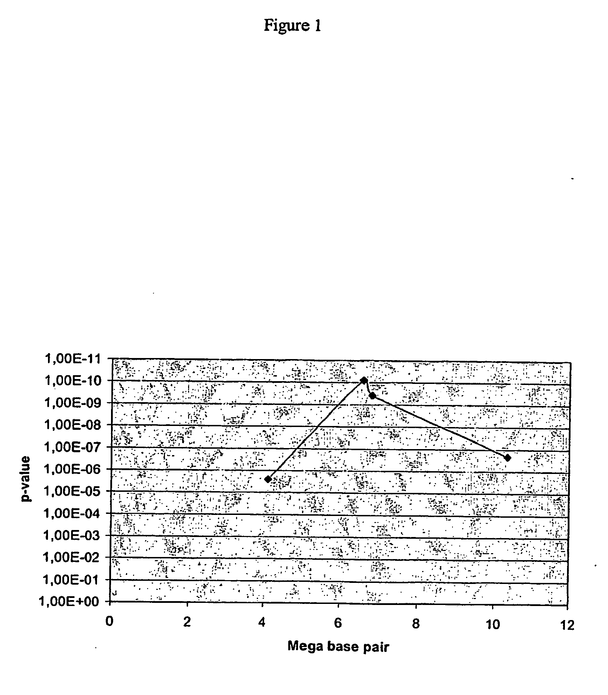

Figure 1 : High density mapping using Genomic Hybrid Identity Profiling (GenomeHIP) A total of 2263 BAC clones with an average spacing of 1.2 Mega base pairs between clones representing the whole human genome were tested for linkage using GenomeHIP. Each point on the x-axis corresponds to a clone. Several clones are indicated by their library name for better orientation (e.g. BACA17ZF07).
Highly significant evidence for linkage was calculated for clones BACA17ZF07 (p-value 8.0x10⁻¹¹) and BACA15ZD05 (p-value 3.8x10⁻¹⁰). Significant evidence for linkage was calculated for clones BACA13ZH10 and BACA13ZH11 (p-value 2.5x10⁶ and 2.1x10⁻⁷, respectively). The whole linkage region is encompassing a region starting from 4126987 base pairs to 7007690 base pairs on human chromosome 1.
The p-value 2x10⁻⁵ corresponding to the significance level for significant linkage was used as a significance level for whole genome screens as proposed by Lander and Kruglyak (1995).

### DETAILED DESCRIPTION OF THE INVENTION

The present invention discloses the identification of KCNAB2 as a human obesity susceptibility gene. Various nucleic acid samples from 164 families with obesity were submitted to a particular GenomeHIP process. This process led to the identification of particular identical-by-descent fragments in said populations that are altered in obese subjects. By screening of the IBD fragments, we identified the potassium voltage-gated channel, shaker-related family, beta member 2 on chromosome 1p36.3 (KCNAB2) gene as a candidate for obesity and related phenotypes. This gene is indeed present in the critical interval and expresses a functional phenotype consistent with a genetic regulation of obesity. SNPs of the KCNAB2 gene were also identified, as being correlated to obesity in human subjects. SNP33 and SNP34 located in the KCNAB2 gene were found to be associated with obesity. Haplotypes disclosed in Table 5 comprising several SNPs selected in the group consisting of SNP2, SNP17, SNP23, SNP33, SNP34, SNP38, SNP42, SNP47, SNP49, SNP51, SNP53 and SNP69 have also been identified as associated with obesity.

The present invention thus proposes to use KCNAB2 gene and corresponding expression products for the diagnosis, prevention and treatment of obesity and associated disorders, as well as for the screening of therapeutically active drugs.

### DEFINITIONS

Obesity and metabolic disorders: Obesity shall be construed as any condition of abnormal or excessive fat accumulation in adipose tissue, to the extent that health may be impaired. Associated disorders, diseases or pathologies include, more specifically, any metabolic disorders, including hypo-alphalipoproteinemia, familial combined hyperlipidemia, insulin resistant syndrome X or multiple metabolic disorder, coronary artery disease, diabetes mellitus and dyslipidemia. The invention may be used in various subjects, particularly human, including adults, children and at the prenatal stage.

Within the context of this invention, the KCNAB2 gene locus designates all KCNAB2 sequences or products in a cell or organism, including KCNAB2 coding sequences, KCNAB2 non-coding sequences (e.g., introns), KCNAB2 regulatory sequences controlling transcription and/or translation (e.g., promoter, enhancer, terminator, etc.), as well as all corresponding expression products, such as KCNAB2 RNAs (e.g., mRNAs) and KCNAB2 polypeptides (e.g., a pre-protein and a mature protein). The KCNAB2 gene locus also comprise surrounding sequences of the KCNAB2 gene which include SNPs that are in linkage disequilibrium with SNPs located in the KCNAB2 gene. For example, the KCNAB2 locus comprises surrounding sequences comprising SNP2, SNP17, SNP23, SNP38, SNP42, SNP47, SNP49, SNP51 and SNP53.

As used in the present application, the term "KCNAB2 gene" designates the potassium voltage-gated channel, shaker-related family, beta member 2 gene on human chromosome 1p36.3, as well as variants, analogs and fragments thereof, including alleles thereof (e.g., germline mutations) which are related to susceptibility to obesity and metabolic disorders. The KCNAB2 gene may also be referred to as AKR6A5, KCNA2B, Hkvbeta2, KVBETA-2, Hkvbeta2.1 and Hkvbeta2.2.

The term "gene" shall be construed to include any type of coding nucleic acid, including gnomic DNA (gDNA), complementary DNA (cDNA), synthetic or semi-synthetic DNA, as well as any form of corresponding RNA. The term gene particularly includes recombinant nucleic acids encoding KCNAB22, i.e., any non naturally occurring nucleic acid molecule created artificially, e.g., by assembling, cutting, ligating or amplifying sequences. A KCNAB2 gene is typically double-stranded, although other forms may be contemplated, such as single-stranded. KCNAB2 genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. Suitable KCNAB2 gene sequences may be found on gene banks, such as Unigene Cluster for KCNAB2 (Hs. 440497) and Unigene Representative Sequences NM_003636 and NM_172130. A particular example of a KCNAB2 gene comprises SEQ ID No: 1 and SEQ ID No: 3.

The term "KCNAB2 gene" includes any variant, fragment or analog of SEQ ID Nos:1 and 3 or of any coding sequence as identified above. Such variants include, for instance, naturally-occurring variants due to allelic variations between individuals (e.g., polymorphisms), mutated alleles related to obesity, alternative splicing forms, etc. The term variant also includes KCNAB2 gene sequences from other sources or organisms. Variants are preferably substantially homologous to SEQ ID Nos:1 and 3, i.e., exhibit a nucleotide sequence identity of at least about 65%, typically at least about 75%, preferably at least about 85%, more preferably at least about 95% with SEQ ID Nos:1 and 3. Variants and analogs of a KCNAB2 gene also include nucleic acid sequences, which hybridize to a sequence as defined above (or a complementary strand thereof) under stringent hybridization conditions.

Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

A fragment of a KCNAB2 gene designates any portion of at least about 8 consecutive nucleotides of a sequence as disclosed above, preferably at least about 15, more preferably at least about 20 nucleotides, further preferably of at least 30 nucleotides. Fragments include all possible nucleotide length between 8 and 100 nucleotides, preferably between 15 and 100, more preferably between 20 and 100.

A KCNAB2 polypeptide designates any protein or polypeptide encoded by a KCNAB2 gene as disclosed above. The term "polypeptide" refers to any molecule comprising a stretch of amino acids. This term includes molecules of various length, such as peptides and proteins. The polypeptide may be modified, such as by glycosylations and/or acetylations and/or chemical reaction or coupling, and may contain one or several non-natural or synthetic amino acids. A specific example of a KCNAB2 polypeptide comprises all or part of SEQ ID No: 2 (NP_003627) and SEQ ID No: 4 (NP_742128) or a variant thereof.

The terms "response to a treatment" refer to treatment efficacy, including but not limited to ability to metabolize a therapeutic compound, to the ability to convert a pro-drug to an active drug, and to the pharmacokinetics (absorption, distribution, elimination) and the pharmacodynamics (receptor-related) of a drug in an individual.

The terms "adverse effects to a treatment" refer to adverse effects of therapy resulting from extensions of the principal pharmacological action of the drug or to idiosyncratic adverse reactions resulting from an interaction of the drug with unique host factors. "Side effects to a treatment" include, but are not limited to, adverse reactions such as dermatologic, hematologic or hepatologic toxicities and further includes gastric and intestinal ulceration, disturbance in platelet function, renal injury, generalized urticaria, bronchoconstriction, hypotension, and shock.

### DIAGNOSIS

The invention now provides diagnosis methods based on a monitoring of the KCNAB2 gene locus in a subject. Within the context of the present invention, the term 'diagnosis" includes the detection, monitoring, dosing, comparison, etc., at various stages, including early, pre-symptomatic stages, and late stages, in adults, children and pre-birth. Diagnosis typically includes the prognosis, the assessment of a predisposition or risk of development, the characterization of a subject to define most appropriate treatment (pharmaco-genetics), etc.

The present invention provides diagnostic methods to determine whether an individual is at risk of developing obesity or an associated disorder or suffers from obesity or an associated disorder resulting from a mutation or a polymorphism in the KCNAB2 gene locus. The present invention also provides methods to determine whether an individual is likely to respond positively to a therapeutic agent or whether an individual is at risk of developing an adverse side effect to a therapeutic agent.

A particular object of this invention resides in a method of detecting the presence of or predisposition to obesity or an associated disorder in a subject, the method comprising detecting in a sample from the subject the presence of an alteration in the KCNAB2 gene locus in said sample. The presence of said alteration is indicative of the presence or predisposition to obesity or an associated disorder. Optionally, said method comprises a previous step of providing a sample from a subject. Preferably, the presence of an alteration in the KCNAB2 gene locus in said sample is detected through the genotyping of a sample.

Another particular object of this invention resides in a method of detecting the protection from obesity or an associated disorder in a subject, the method comprising detecting the presence of an alteration in the KCNAB2 gene locus in a sample from the subject, the presence of said alteration being indicative of the protection from obesity or an associated disorder.

In a preferred embodiment, said alteration is one or several SNP(s) or a haplotype of SNPs associated with obesity. More preferably, said haplotype associated with obesity comprises or consists of several SNPs selected in the group consisting of SNP2, SNP17, SNP23, SNP33, SNP34, SNP38, SNP42, SNP47, SNP49, SNP51, SNP53 and SNP69. Still more preferably, said haplotype is selected from the haplotypes disclosed in Table 5. More preferably, said SNP associated with obesity can be SNP33 or SNP34.

Another particular object of this invention resides in a method of assessing the response of a subject to a treatment of obesity or an associated disorder, the method comprising (i) providing a sample from the subject and (ii) detecting the presence of an alteration in the KCNAB2 gene locus in said sample.

Another particular object of this invention resides in a method of assessing the response of a subject to a treatment of obesity or an associated disorder, the method comprising detecting in a sample from the subject the presence of an alteration in the KCNAB2 gene locus in said sample. The presence of said alteration is indicative of a particular response to said treatment. Preferably, the presence of an alteration in the KCNAB2 gene locus in said sample is detected through the genotyping of a sample.

A further particular object of this invention resides in a method of assessing the adverse effects of a subject to a treatment of obesity or an associated disorder, the method comprising detecting in a sample from the subject the presence of an alteration in the KCNAB2 gene locus in said sample. The presence of said alteration is indicative of adverse effects to said treatment. Preferably, the presence of an alteration in the KCNAB2 gene locus in said sample is detected through the genotyping of a sample.

In a preferred embodiment, said alteration is one or several SNP(s) or a haplotype of SNPs associated with obesity. More preferably, said haplotype associated with obesity comprises or consists of several SNPs selected in the group consisting of SNP2, SNP17, SNP23, SNP33, SNP34, SNP38, SNP42, SNP47, SNP49, SNP51, SNP53 and SNP69. Still more preferably, said haplotype is selected from the haplotypes disclosed in Table 5. More preferably, said SNP associated with obesity can be SNP33 or SNP34.

In an additional embodiment, the invention concerns a method for preventing obesity or an associated disorder in a subject, comprising detecting the presence of an alteration in the KCNAB2 gene locus in a sample from the subject, the presence of said alteration being indicative of the predisposition to obesity or an associated disorder, and, administering a prophylactic treatment against obesity or an associated disorder. Said prophylactic treatment can be an administration of a drug and/or a diet.

Diagnostics, which analyze and predict response to a treatment or drug, or side effects to a treatment or drug, may be used to determine whether an individual should be treated with a particular treatment drug. For example, if the diagnostic indicates a likelihood that an individual will respond positively to treatment with a particular drug, the drug may be administered to the individual. Conversely, if the diagnostic indicates that an individual is likely to respond negatively to treatment with a particular drug, an alternative course of treatment may be prescribed. A negative response may be defined as either the absence of an efficacious response or the presence of toxic side effects.

Clinical drug trials represent another application for the KCNAB2 SNPs. One or more KCNAB2 SNPs indicative of response to a drug or to side effects to a drug may be identified using the methods described above. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

The alteration may be determined at the level of the KCNAB2 gDNA, RNA or polypeptide. Optionally, the detection is performed by sequencing all or part of the KCNAB2 gene or by selective hybridisation or amplification of all or part of the KCNAB2 gene. More preferably a KCNAB2 gene specific amplification is carried out before the alteration identification step.

An alteration in the KCNAB2 gene locus may be any form of mutation(s), deletion(s), rearrangement(s) and/or insertions in the coding and/or non-coding region of the locus, alone or in various combination(s). Mutations more specifically include point mutations. Deletions may encompass any region of two or more residues in a coding or non-coding portion of the gene locus, such as from two residues up to the entire gene or locus. Typical deletions affect smaller regions, such as domains (introns) or repeated sequences or fragments of less than about 50 consecutive base pairs, although larger deletions may occur as well. Insertions may encompass the addition of one or several residues in a coding or non-coding portion of the gene locus. Insertions may typically comprise an addition of between 1 and 50 base pairs in the gene locus. Rearrangement includes inversion of sequences. The KCNAB2 gene locus alteration may result in the creation of stop codons, frameshift mutations, amino acid substitutions, particular RNA splicing or processing, product instability, truncated polypeptide production, etc. The alteration may result in the production of a KCNAB2 polypeptide with altered function, stability, targeting or structure. The alteration may also cause a reduction in protein expression or, alternatively, an increase in said production.

In a particular embodiment of the method according to the present invention, the alteration in the KCNAB2 gene locus is selected from a point mutation, a deletion and an insertion in the KCNAB2 gene or corresponding expression product, more preferably a point mutation and a deletion. The alteration may be determined at the level of the KCNAB2 gDNA, RNA or polypeptide.

In this regard, the present invention now discloses two SNPs in the KCNAB2 gene and certain haplotypes characterized by one of the two above SNPs which are associated with obesity. The SNPs are reported in the following table 2.

**Table 2**

| Nucleotide position in genomic sequence of chromosome 1 (Build34) | SNP identity | dbSNP reference | Polymorphism | Position in locus and type of amino acid change | Sequence reference |
|---|---|---|---|---|---|
| 4356069 | SNP2 | rs875806 | G/C | 5' of KCNAB2 locus | SEQ ID No 5 |
| 5054675 | SNP17 | Not available | G/A | 5' of KCAB2 locus | SEQ ID No 6 |
| 5634292 | SNP23 | rs1287635 | T/C | 5' of KCAB2 locus | SEQ ID No 7 |
| 5863071 | SNP33 | rs3789529 | A/G | intron | SEQ ID No 8 |
| 5868108 | SNP34 | rs3747977 | G/A | Coding, synonymous | SEQ ID No 9 |
| 6028263 | SNP38 | rs3747977 | G/A | 3' of KCNAB2 locus | SEQ ID No 10 |
| 6120760 | SNP42 | rs722333 | A/G | 3' of KCNAB2 locus | SEQ ID No 11 |
| 6272613 | SNP47 | rs3007434 | G/A | 3' of KCNAB2 locus | SEQ ID No 12 |
| 6354269 | SNP49 | rs731024 | G/A | 3' of KCNAB2 locus | SEQ ID No 13 |
| 6366970 | SNP51 | Not available | C/A | 3' of KCNAB2 locus | SEQ ID No 14 |
| 6394452 | SNP53 | rs2076363 | G/C | 3' of KCNAB2 locus | SEQ ID No 15 |
| 7112026 | SNP69 | rs705681 | T/C | 3' of KCNAB2 locus | SEQ ID No 16 |

In another variant, the method comprises detecting the presence of an altered KCNAB2 RNA expression. Altered RNA expression includes the presence of an altered RNA sequence, the presence of an altered RNA splicing or processing, the presence of an altered quantity of RNA, etc. These may be detected by various techniques known in the art, including by sequencing all or part of the KCNAB2 RNA or by selective hybridisation or selective amplification of all or part of said RNA, for instance.

In a further variant, the method comprises detecting the presence of an altered KCNAB2 polypeptide expression. Altered KCNAB2 polypeptide expression includes the presence of an altered polypeptide sequence, the presence of an altered quantity of KCNAB2 polypeptide, the presence of an altered tissue distribution, etc. These may be detected by various techniques known in the art, including by sequencing and/or binding to specific ligands (such as antibodies), for instance.

As indicated above, various techniques known in the art may be used to detect or quantify altered KCNAB2 gene or RNA expression or sequence, including sequencing, hybridisation, amplification and/or binding to specific ligands (such as antibodies). Other suitable methods include allele-specific oligonucleotide (ASO), allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, heteroduplex analysis, RNase protection, chemical mismatch cleavage, ELISA, radio-immunoassays (RIA) and immuno-enzymatic assays (IEMA).

Some of these approaches (e.g., SSCA and CGGE) are based on a change in electrophoretic mobility of the nucleic acids, as a result of the presence of an altered sequence. According to these techniques, the altered sequence is visualized by a shift in mobility on gels. The fragments may then be sequenced to confirm the alteration.

Some others are based on specific hybridisation between nucleic acids from the subject and a probe specific for wild-type or altered KCNAB2 gene or RNA. The probe may be in suspension or immobilized on a substrate. The probe is typically labelled to facilitate detection of hybrids.

Some of these approaches are particularly suited for assessing a polypeptide sequence or expression level, such as Northern blot, ELISA and RIA. These latter require the use of a ligand specific for the polypeptide, more preferably of a specific antibody.

In a particular, preferred, embodiment, the method comprises detecting the presence of an altered KCNAB2 gene expression profile in a sample from the subject. As indicated above, this can be accomplished more preferably by sequencing, selective hybridisation and/or selective amplification of nucleic acids present in said sample.

### Sequencing

Sequencing can be carried out using techniques well known in the art, using automatic sequencers. The sequencing may be performed on the complete KCNAB2 gene or, more preferably, on specific domains thereof, typically those known or suspected to carry deleterious mutations or other alterations.

### Amplification

Amplification is based on the formation of specific hybrids between complementary nucleic acid sequences that serve to initiate nucleic acid reproduction.

Amplification may be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the KCNAB2 gene or locus are able to specifically hybridize with a portion of the KCNAB2 gene locus that flank a target region of said locus, said target region being altered in certain subjects having obesity or associated disorders. Examples of such target regions are provided in Table 2.

Primers that can be used to amplify KCNAB2 target region comprising SNPs as identified in Table 2 may be designed based on the sequence of SEQ ID NO: 1 or 3 or on the genomic sequence of KCNAB2.

Another particular object of this invention resides in a nucleic acid primer useful for amplifying sequences from the KCNAB2 gene or locus including surrounding regions. Such primers are preferably complementary to, and hybridize specifically to nucleic acid sequences in the KCNAB2 gene locus. Particular primers are able to specifically hybridise with a portion of the KCNAB2 gene locus that flank a target region of said locus, said target region being altered in certain subjects having obesity or associated disorders.

The invention also relates to a nucleic acid primer, said primer being complementary to and hybridizing specifically to a portion of a KCNAB2 coding sequence (e.g., gene or RNA) altered in certain subjects having obesity or associated disorders. In this regard, particular primers of this invention are specific for altered sequences in a KCNAB2 gene or RNA. By using such primers, the detection of an amplification product indicates the presence of an alteration in the KCNAB2 gene locus. In contrast, the absence of amplification product indicates that the specific alteration is not present in the sample.

Typical primers of this invention are single-stranded nucleic acid molecules of about 5 to 60 nucleotides in length, more preferably of about 8 to about 25 nucleotides in length. The sequence can be derived directly from the sequence of the KCNAB2 gene locus. Perfect complementarity is preferred, to ensure high specificity. However, certain mismatch may be tolerated.

The invention also concerns the use of a nucleic acid primer or a pair of nucleic acid primers as described above in a method of detecting the presence of or predisposition to obesity or an associated disorder in a subject or in a method of assessing the response of a subject to a treatment of obesity or an associated disorder.

### Selective hybridization

Hybridization detection methods are based on the formation of specific hybrids between complementary nucleic acid sequences that serve to detect nucleic acid sequence alteration(s).

A particular detection technique involves the use of a nucleic acid probe specific for wild-type or altered KCNAB2 gene or RNA, followed by the detection of the presence of a hybrid. The probe may be in suspension or immobilized on a substrate or support (as in nucleic acid array or chips technologies). The probe is typically labelled to facilitate detection of hybrids.

In this regard, a particular embodiment of this invention comprises contacting the sample from the subject with a nucleic acid probe specific for an altered KCNAB2 gene locus, and assessing the formation of an hybrid. In a particular, preferred embodiment, the method comprises contacting simultaneously the sample with a set of probes that are specific, respectively, for wild type KCNAB2 gene locus and for various altered forms thereof. In this embodiment, it is possible to detect directly the presence of various forms of alterations in the KCNAB2 gene locus in the sample. Also, various samples from various subjects may be treated in parallel.

Within the context of this invention, a probe refers to a polynucleotide sequence which is complementary to and capable of specific hybridisation with a (target portion of a) KCNAB2 gene or RNA, and which is suitable for detecting polynucleotide polymorphisms associated with KCNAB2 alleles which predispose to or are associated with obesity or metabolic disorders. Probes are preferably perfectly complementary to the KCNAB2 gene, RNA, or target portion thereof. Probes typically comprise single-stranded nucleic acids of between 8 to 1000 nucleotides in length, for instance of between 10 and 800, more preferably of between 15 and 700, typically of between 20 and 500. It should be understood that longer probes may be used as well. A preferred probe of this invention is a single stranded nucleic acid molecule of between 8 to 500 nucleotides in length, which can specifically hybridise to a region of a KCNAB2 gene or RNA that carries an alteration.

A specific embodiment of this invention is a nucleic acid probe specific for an altered (e.g., a mutated) KCNAB2 gene or RNA, i.e., a nucleic acid probe that specifically hybridises to said altered KCNAB2 gene or RNA and essentially does not hybridise to a KCNAB2 gene or RNA lacking said alteration. Specificity indicates that hybridisation to the target sequence generates a specific signal which can be distinguished from the signal generated through non-specific hybridisation. Perfectly complementary sequences are preferred to design probes according to this invention. It should be understood, however, that certain mismatch may be tolerated, as long as the specific signal may be distinguished from non-specific hybridisation.

Particular examples of such probes are nucleic acid sequences complementary to a target portion of the genomic region including the KCNAB2 gene or RNA carrying a point mutation as listed in Table 2 above. More particularly, the probes can comprise a sequence selected from the group consisting of SEQ ID Nos 5-16 or a fragment thereof comprising the SNP or a complementary sequence thereof.

The sequence of the probes can be derived from the sequences of the KCNAB2 gene and RNA as provided in the present application. Nucleotide substitutions may be performed, as well as chemical modifications of the probe. Such chemical modifications may be accomplished to increase the stability of hybrids (e.g., intercalating groups) or to label the probe. Typical examples of labels include, without limitation, radioactivity, fluorescence, luminescence, enzymatic labelling, etc.

The invention also concerns the use of a nucleic acid probe as described above in a method of detecting the presence of or predisposition to obesity or an associated disorder in a subject or in a method of assessing the response of a subject to a treatment of obesity or an associated disorder.

### Specific Ligand Binding

As indicated above, alteration in the KCNAB2 gene locus may also be detected by screening for alteration(s) in KCNAB2 polypeptide sequence or expression levels. In this regard, a specific embodiment of this invention comprises contacting the sample with a ligand specific for a KCNAB2 polypeptide and determining the formation of a complex.

Different types of ligands may be used, such as specific antibodies. In a specific embodiment, the sample is contacted with an antibody specific for a KCNAB2 polypeptide and the formation of an immune complex is determined. Various methods for detecting an immune complex can be used, such as ELISA, radio-immunoassays (RIA) and immuno-enzymatic assays (IEMA).

Within the context of this invention, an antibody designates a polyclonal antibody, a monoclonal antibody, as well as fragments or derivatives thereof having substantially the same antigen specificity. Fragments include Fab, Fab'2, CDR regions, etc. Derivatives include single-chain antibodies, humanized antibodies, poly-functional antibodies, etc.

An antibody specific for a KCNAB2 polypeptide designates an antibody that selectively binds a KCNAB2 polypeptide, i.e., an antibody raised against a KCNAB2 polypeptide or an epitope-containing fragment thereof. Although non-specific binding towards other antigens may occur, binding to the target KCNAB2 polypeptide occurs with a higher affinity and can be reliably discriminated from non-specific binding.

In a specific embodiment, the method comprises contacting a sample from the subject with (a support coated with) an antibody specific for an altered form of a KCNAB2 polypeptide, and determining the presence of an immune complex. In a particular embodiment, the sample may be contacted simultaneously, or in parallel, or sequentially, with various (supports coated with) antibodies specific for different forms of a KCNAB2 polypeptide, such as a wild-type and various altered forms thereof.

The invention also concerns the use of a ligand, preferably an antibody, a fragment or a derivative thereof as described above, in a method of detecting the presence of or predisposition to obesity or associated disorders in a subject or in a method of assessing the response of a subject to a treatment of obesity or associated disorders.

The invention also relates to a diagnostic kit comprising products and reagents for detecting in a sample from a subject the presence of an alteration in the KCNAB2 gene or polypeptide, in the KCNAB2 gene or polypeptide expression, and/or in KCNAB2 activity. Said diagnostic kit according to the present invention comprises any primer, any pair of primers, any nucleic acid probe and/or any ligand, preferably antibody, described in the present invention. Said diagnostic kit according to the present invention can further comprise reagents and/or protocols for performing a hybridization, amplification or antigen-antibody immune reaction.

The diagnosis methods can be performed in vitro, ex vivo or in vivo, preferably in vitro or ex vivo. They use a sample from the subject, to assess the status of the KCNAB2 gene locus. The sample may be any biological sample derived from a subject, which contains nucleic acids or polypeptides. Examples of such samples include fluids, tissues, cell samples, organs, biopsies, etc. Most preferred samples are blood, plasma, saliva, urine, seminal fluid, etc. Pre-natal diagnosis may also be performed by testing foetal cells or placental cells, for instance The sample may be collected according to conventional techniques and used directly for diagnosis or stored. The sample may be treated prior to performing the method, in order to render or improve availability of nucleic acids or polypeptides for testing. Treatments include, for instant, lysis (e.g., mechanical, physical, chemical, etc.), centrifugation, etc. Also, the nucleic acids and/or polypeptides may be prepurified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

As indicated, the sample is preferably contacted with reagents such as probes, primers or ligands in order to assess the presence of an altered KCNAB2 gene locus. Contacting may be performed in any suitable device, such as a plate, tube, well, glass, etc. In specific embodiments, the contacting is performer on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

The finding of an altered KCNAB2 polypeptide, RNA or DNA in the sample is indicative of the presence of an altered KCNAB2 gene locus in the subject, which can be correlated to the presence, predisposition or stage of progression of obesity or metabolic disorders.

For example, an individual having a germline KCNAB2 mutation has an increased risk of developing obesity or metabolic disorders. The determination of the presence of an altered KCNAB2 gene locus in a subject also allows the design of appropriate therapeutic intervention, which is more effective and customized. Also, this determination at the pre-symptomatic level allows a preventive regimen to be applied.

### Linkage Disequilibirum

Once a first SNP has been identified in a genomic region of interest, more particularly in KCNAB2 gene locus, the practitioner of ordinary skill in the art can easily identify additional SNPs in linkage disequilibrium with this first SNP. Indeed, any SNP in linkage disequilibrium with a first SNP associated with obesity or an associated metabolic disorder will be associated with this trait. Therefore, once the association has been demonstrated between a given SNP and obesity or an associated metabolic disorder, the discovery of additional SNPs associated with this trait can be of great interest in order to increase the density of SNPs in this particular region.

Identification of additional SNPs in linkage disequilibrium with a given SNP involves: (a) amplifying a fragment from the genomic region comprising or surrounding a first SNP from a plurality of individuals; (b) identifying of second SNPs in the genomic region harboring or surrounding said first SNP; (c) conducting a linkage disequilibrium analysis between said first SNP and second SNPs; and (d) selecting said second SNPs as being in linkage disequilibrium with said first marker. Subcombinations comprising steps (b) and (c) are also contemplated.

Methods to identify SNPs and to conduct linkage disequilibrium analysis can be carried out by the skilled person without undue experimentation by using well-known methods.

These SNPs in linkage disequilibrium can also be used in the methods according to the present invention, and more particularly in the diagnosic methods according to the present invention.

For example, a linkage locus of Crohn's disease has been mapped to a large region spanning 18cM on chromosome 5q31 (Rioux et al., 2000 and 2001). Using dense maps of microsatellite markers and SNPs across the entire region, strong evidence of linkage disequilibrium (LD) was found. Having found evidence of LD, the authors developed an ultra-high-density SNP map and studied a denser collection of markers selected from this map. Multilocus analyses defined a single common risk haplotype characterised by multiple SNPs that were each independently associated using TDT. These SNPs were unique to the risk haplotype and essentially identical in their information content by virtue of being in nearly complete LD with one another. The equivalent properties of these SNPs make it impossible to identify the causal mutation within this region on the basis of genetic evidence alone.

### Causal Mutation

Mutations in the KCNAB2 gene which are responsible for obesity or an associated metabolic disorder may be identified by comparing the sequences of the KCNAB2 gene from patients presenting obesity or an associated metabolic disorder and control individuals. Based on the identified association of SNPs of KCNAB2 and obesity or an associated metabolic disorder, the identified locus can be scanned for mutations. In a preferred embodiment, functional regions such as exons and splice sites, promoters and other regulatory regions of the KCNAB2 gene are scanned for mutations. Preferably, patients presenting obesity or an associated metabolic disorder carry the mutation shown to be associated with obesity or an associated metabolic disorder and controls individuals do not carry the mutation or allele associated with obesity or an associated metabolic disorder. It might also be possible that patients presenting obesity or an associated metabolic disorder carry the mutation shown to be associated with obesity or an associated metabolic disorder with a higher frequency than controls individuals.

The method used to detect such mutations generally comprises the following steps: amplification of a region of the KCNAB2 gene comprising a SNP or a group of SNPs associated with obesity or an associated metabolic disorder from DNA samples of the KCNAB2 gene from patients presenting obesity or an associated metabolic disorder and control individuals; sequencing of the amplified region; comparison of DNA sequences of the KCNAB2 gene from patients presenting obesity or an associated metabolic disorder and control individuals; determination of mutations specific to patients presenting obesity or an associated metabolic disorder.

Therefore, identification of a causal mutation in the KCNAB2 gene can be carried out by the skilled person without undue experimentation by using well-known methods.

For example, the causal mutations have been identified in the following examples by using routine methods.

Hugot et al. (2001) applied a positional cloning strategy to identify gene variants with susceptibly to Crohn's disease in a region of chromosome 16 previously found to be linked to susceptibility to Crohn's disease. To refine the location of the potential sucecptibility locus 26 microsatellite markers were genotyped and tested for association to Crohn's disease using the transmission disequilibrium test. A borderline significant association was found between one allele of the microsatellite marker D16S136. Eleven additional SNPs were selected from surrounding regions and several SNPs showed significant association. SNP5-8 from this region were found to be present in a single exon of the NOD2/CARD 15 gene and shown to be non-synonymous variants. This prompted the authors to sequence the complete coding sequence of this gene in 50 CD patients. Two additional non-synonymous mutations (SNP12 and SNP13) were found. SNP13 was most significant associated (p=6x10-6) using the pedigree transmission disequilibrium test. In another independent study, the same variant was found also by sequencing the coding region of this gene from 12 affected individuals compared to 4 controls (Ogura et al., 2001). The rare allele of SNP13 corresponded to a 1-bp insertion predicted to truncate the NOD2/CARD15 protein. This allele was also present in normal healthy individuals, albeit with significantly lower frequency as compared to the controls.

Similarly, Lesage et al. (2002) performed a mutational analyses of CARD15 in 453 patients with CD, including 166 sporadic and 287 familial cases, 159 patients with ulcerative colitis (UC), and 103 healthy control subjects by systematic sequencing of the coding region. Of 67 sequence variations identified, 9 had an allele frequency >5% in patients with CD. Six of them were considered to be polymorphisms, and three (SNP12-R702W, SNP8-G908R, and SNP13-1007fs) were confirmed to be independently associated with susceptibility to CD. Also considered as potential disease-causing mutations (DCMs) were 27 rare additional mutations. The three main variants (R702W, G908R, and 1007fs) represented 32%, 18%, and 31%, respectively, of the total CD mutations, whereas the total of the 27 rare mutations represented 19% of DCMs. Altogether, 93% of the mutations were located in the distal third of the gene. No mutations were found to be associated with UC. In contrast, 50% of patients with CD carried at least one DCM, including 17% who had a double mutation.

### DRUG SCREENING

The present invention also provides novel targets and methods for the screening of drug candidates or leads. The methods include binding assays and/or functional assays, and may be performed in vitro, in cell systems, in animals, etc.

A particular object of this invention resides in a method of selecting biologically active compounds, said method comprising contacting in vitro a test compound with a KCNAB2 gene or polypeptide according to the present invention and determining the ability of said test compound to bind said KCNAB2 gene or polypeptide. Binding to said gene or polypeptide provides an indication as to the ability of the compound to modulate the activity of said target, and thus to affect a pathway leading to obesity or metabolic disorders in a subject. In a preferred embodiment, the method comprises contacting in vitro a test compound with a KCNAB2 polypeptide or a fragment thereof according to the present invention and determining the ability of said test compound to bind said KCNAB2 polypeptide or fragment. The fragment preferably comprises a binding site of the KCNAB2 polypeptide. Preferably, said KCNAB2 gene or polypeptide or a fragment thereof is an altered or mutated KCNAB2 gene or polypeptide or a fragment thereof comprising the alteration or mutation.

A particular object of this invention resides in a method of selecting compounds active on obesity and associated disorders, said method comprising contacting in vitro a test compound with a KCNAB2 polypeptide according to the present invention or binding site-containing fragment thereof and determining the ability of said test compound to bind said KCNAB2 polypeptide or fragment thereof. Preferably, said KCNAB2 polypeptide or a fragment thereof is an altered or mutated KCNAB2 polypeptide or a fragment thereof comprising the alteration or mutation.

In a further particular embodiment, the method comprises contacting a recombinant host cell expressing a KCNAB2 polypeptide according to the present invention with a test compound, and determining the ability of said test compound to bind said KCNAB2 and to modulate the activity of KCNAB2 polypeptide. Preferably, said KCNAB2 polypeptide or a fragment thereof is an altered or mutated KCNAB2 polypeptide or a fragment thereof comprising the alteration or mutation.

The determination of binding may be performed by various techniques, such as by labelling of the test compound, by competition with a labelled reference ligand, etc.

A further object of this invention resides in a method of selecting biologically active compounds, said method comprising contacting in vitro a test compound with a KCNAB2 polypeptide according to the present invention and determining the ability of said test compound to modulate the activity of said KCNAB2 polypeptide. Preferably, said KCNAB2 polypeptide or a fragment thereof is an altered or mutated KCNAB2 polypeptide or a fragment thereof comprising the alteration or mutation.

A further object of this invention resides in a method of selecting biologically active compounds, said method comprising contacting in vitro a test compound with a KCNAB2 gene according to the present invention and determining the ability of said test compound to modulate the expression of said KCNAB2 gene. Preferably, said KCNAB2 gene or a fragment thereof is an altered or mutated KCNAB2 gene or a fragment thereof comprising the alteration or mutation.

In an other embodiment, this invention relates to a method of screening, selecting or identifying active compounds, particularly compounds active on obesity or metabolic disorders, the method comprising contacting a test compound with a recombinant host cell comprising a reporter construct, said reporter construct comprising a reporter gene under the control of a KCNAB2 gene promoter, and selecting the test compounds that modulate (e.g. stimulate or reduce) expression of the reporter gene. Preferably, said KCNAB2 gene promoter or a fragment thereof is an altered or mutated KCNAB2 gene promoter or a fragment thereof comprising the alteration or mutation.

In a particular embodiment of the methods of screening, the modulation is an inhibition. In an other particular embodiment of the methods of screening, the modulation is an activation.

The above screening assays may be performed in any suitable device, such as plates, tubes, dishes, flasks, etc. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

### PHARMACEUTICAL COMPOSITIONS, THERAPY

A further object of this invention is a pharmaceutical composition comprising (i) a KCNAB2 polypeptide or a fragment thereof, a nucleic acid encoding a KCNAB2 polypeptide or a fragment thereof, a vector or a recombinant host cell as described above and (ii) a pharmaceutically acceptable carrier or vehicle.

The invention also relates to a method of treating or preventing obesity or an associated disorder in a subject, the method comprising administering to said subject a functional (e.g., wild-type) KCNAB2 polypeptide or a nucleic acid encoding the same.

An other embodiment of this invention resides in a method of treating or preventing obesity or an associated disorder in a subject, the method comprising administering to said subject a compound that modulates, preferably that activates or mimics, expression or activity of a KCNAB2 gene or protein according to the present invention. Said compound can be an agonist or an antagonist of KCNAB2, an antisense or a RNAi of KCNAB2, an antibody or a fragment or a derivative thereof specific to a KCNAB2 polypeptide according to the present invention. In a particular embodiment of the method, the modulation is an inhibition. In an other particular embodiment of the method, the modulation is an activation.

The invention also relates, generally, to the use of a functional KCNAB2 polypeptide, a nucleic acid encoding the same, or a compound that modulates expression or activity of a KCNAB2 gene or protein according to the present invention, in the manufacture of a pharmaceutical composition for treating or preventing obesity or an associated metabolic disorder in a subject. Said compound can be an agonist or an antagonist of KCNAB2, an antisense or a RNAi of KCNAB2, an antibody or a fragment or a derivative thereof specific to a KCNAB2 polypeptide according to the present invention. In a particular embodiment of the method, the modulation is an inhibition. In an other particular embodiment of the method, the modulation is an activation.

The present invention demonstrates the correlation between obesity (and related disorders) and the KCNAB2 gene locus. The invention thus provides a novel target of therapeutic intervention. Various approaches can be contemplated to restore or modulate the KCNAB2 activity or function in a subject, particularly those carrying an altered KCNAB2 gene locus. Supplying wild-type function to such subjects is expected to suppress phenotypic expression of obesity and associated disorders in a pathological cell or organism. The supply of such function can be accomplished through gene or protein therapy, or by administering compounds that modulate or mimic KCNAB2 polypeptide activity (e.g., agonists as identified in the above screening assays).

The wild-type KCNAB2 gene or a functional part thereof may be introduced into the cells of the subject in need thereof using a vector as described above. The vector may be a viral vector or a plasmid. The gene may also be introduced as naked DNA. The gene may be provided so as to integrate into the genome of the recipient host' cells, or to remain extra-chromosomal. Integration may occur randomly or at precisely defined sites, such as through homologous recombination. In particular, a functional copy of the KCNAB2 gene may be inserted in replacement of an altered version in a cell, through homologous recombination. Further techniques include gene gun, liposome-mediated transfection, cationic lipid-mediated transfection, etc. Gene therapy may be accomplished by direct gene injection, or by administering ex vivo prepared genetically modified cells expressing a functional KCNAB2 polypeptide.

Other molecules with KCNAB2 activity (e.g., peptides, drugs, KCNAB2 agonists, or organic compounds) may also be used to restore functional KCNAB2 activity in a subject or to suppress the deleterious phenotype in a cell.

Restoration of functional KCNAB2 gene function in a cell may be used to prevent the development of obesity or metabolic disorders or to reduce progression of said diseases.

Such a treatment may suppress the obese phenotype of a cell, particularly those cells carrying a deleterious allele.

Further aspects and advantages of the present invention will be disclosed in the following experimental section, which should be regarded as illustrative and not limiting the scope of the present application.

### GENE, VECTORS, RECOMBINANT CELLS AND POLYPEPTIDES

A further aspect of this invention resides in novel products for use in diagnosis, therapy or screening. These products comprise nucleic acid molecules encoding a KCNAB2 polypeptide or a fragment thereof, vectors comprising the same, recombinant host cells and expressed polypeptides.

More particularly, the invention concerns an altered or mutated KCNAB2 gene or a fragment thereof comprising said alteration or mutation. The invention also concerns nucleic acid molecules encoding an altered or mutated KCNAB2 polypeptide or a fragment thereof comprising said alteration or mutation. Said alteration or mutation modifies the KCNAB2 activity. The modified activity can be increased or decreased. The invention further concerns a vector comprising an altered or mutated KCNAB2 gene or a fragment thereof comprising said alteration or mutation or a nucleic acid molecule encoding an altered or mutated KCNAB2 polypeptide or a fragment thereof comprising said alteration or mutation, recombinant host cells and expressed polypeptides.

A further object of this invention is a vector comprising a nucleic acid encoding a KCNAB2 polypeptide according to the present invention. The vector may be a cloning vector or, more preferably, an expression vector, i.e., a vector comprising regulatory sequences causing expression of a KCNAB2 polypeptide from said vector in a competent host cell.

These vectors can be used to express a KCNAB2 polypeptide in vitro, ex vivo or in vivo, to create transgenic or "Knock Out" non-human animals, to amplify the nucleic acids, to express antisense RNAs, etc.

The vectors of this invention typically comprise a KCNAB2 coding sequence according to the present invention operably linked to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses, adenoviruses, AAVs, etc., according to recombinant DNA techniques known in the art.

In this regard, a particular object of this invention resides in a recombinant virus encoding a KCNAB2 polypeptide as defined above. The recombinant virus is preferably replication-defective, even more preferably selected from E1- and/or E4-defective adenoviruses, Gag-, pol- and/or env-defective retroviruses and Rep- and/or Cap-defective AAVs. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

A further object of the present invention resides in a recombinant host cell comprising a recombinant KCNAB2 gene or a vector as defined above. Suitable host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

The present invention also relates to a method for producing a recombinant host cell expressing a KCNAB2 polypeptide according to the present invention, said method comprising (i) introducing in vitro or ex vivo into a competent host cell a recombinant nucleic acid or a vector as described above, (ii) culturing in vitro or ex vivo the recombinant host cells obtained and (iii), optionally, selecting the cells which express the KCNAB2 polypeptide.

Such recombinant host cells can be used for the production of KCNAB2 polypeptides, as well as for screening of active molecules, as described below. Such cells may also be used as a model system to study obesity and metabolic disorders. These cells can be maintained in suitable culture media, such as DMEM, RPMI, HAM, etc., in any appropriate culture device (plate, flask, dish, tube, pouch, etc.).

### EXAMPLES

### 1. Identification of an Obesity susceptibility locus on human chromosome 1

### A. GenomeHIP platform to identify the chromosome 1 susceptibility gene

The GenomeHIP platform was applied to allow rapid identification of an obesity susceptibility gene.
Briefly, the technology consists of forming pairs from the DNA of related individuals. Each DNA is marked with a specific label allowing its identification. Hybrids are then formed between the two DNAs. A particular process (WO00/53802) is then applied that selects all fragments identical-by-descent (IBD) from the two DNAs in a multi step procedure. The remaining IBD enriched DNA is then scored against a BAC clone derived DNA microarray that allows the positioning of the IBD fraction on a chromosome.

The application of this process over many different families results in a matrix of IBD fractions for each pair from each family. Statistical analyses then calculate the minimal IBD regions that are shared between all families tested. Significant results (p-values) are evidence for linkage of the positive region with the trait of interest (here obesity). The linked interval can be delimited by the two most distant clones showing significant p-values.

In the present study, 164 families of German origin (178 independent sib-pairs) concordant for massive obesity (as defined by a body mass index > 90th%ile) were submitted to the GenomeHIP process. The resulting IBD enriched DNA fractions were then labelled with Cy5 fluorescent dyes and hybridised against a DNA array consisting of 2263 BAC clones covering the whole human genome with an average spacing of 1.2 Mega base pairs. Non-selected DNA labelled with Cy3 was used to normalize the signal values and compute ratios for each clone. Clustering of the ratio results was then performed to determine the IBD status for each clone and pair.

By applying this procedure, several BAC clones (BACA13ZH10, BACA17ZF07 and BACA15ZD05) spanning approximately 3 Mega bases in the region on chromosome 1 (bases 4126987 to 7007690) were identified, that showed significant evidence for linkage to obesity (p< 2.5x10⁻⁵).

Table 3: Linkage results for chromosome 1 in the KCNAB2 locus: Indicated is the region correspondent to 3 BAC clones with evidence for linkage. The start and stop positions of the clones correspond to their genomic location based on NCBI Build34 sequence respective to the start of the chromosome (p-ter).

**Table 3**

| Human chromosome | Clones | Start | End | Proportion of informative pairs | p-value |
|---|---|---|---|---|---|
| 1 | BACA13ZH10 | | 4 126 987 | 0.92 | 2.50E-06 |
| 1 | BACA17ZF07 | 6 589 325 | 6 686 208 | 0.94 | 8.00E-11 |
| 1 | BACA15ZD05 | 6 817 039 | 7007690 | 0.93 | 3.80E-10 |

### B. Identification of an obesity susceptibility gene on chromosome 1

By screening the aforementioned 3 Mega bases in the linked chromosomal region, we identified the potassium voltage-gated channel, shaker-related family, beta member 2 (KCNAB2) gene as a candidate for obesity and related phenotypes. This gene is indeed present in the critical interval, with evidence for linkage delimited by the clones outlined above.

KCNAB2 gene encodes a predicted 367-amino acid polypeptide for NP_003627 (mRNA NM_003636 3.17 kb) and spreads over 59.9 kb of genomic sequence. The protein encoded by the gene is a member of the potassium channel, voltage-gated, shaker-related subfamily. This member is one of the beta subunits, which are auxiliary proteins associating with functional Kv-alpha subunits. This member alters functional properties of the KCNA4 gene product (Kv1.4 alpha subunit). Alternative splicing of this gene results in an other transcript variant (NM_172130) encoding a distinct isoform (NP_742128).

Voltage-gated potassium (Kv) channels represent the most complex class of voltage-gated ion channels from both functional and structural standpoints. Their diverse functions include regulating neurotransmitter release, heart rate, insulin secretion, neuronal excitability, epithelial electrolyte transport, smooth muscle contraction, and cell volume.

Recent investigations suggest that Kv channels are active participants in the regulation of beta-cell electrical activity and insulin secretion (MacDonald and Wheeler, 2003). Beta-cell Kv channels are also targets of the G-protein coupled GLP-1 receptor and signals from glucose metabolism, pathways which could be physiologically relevant to the control of insulin secretion.

Examination of Kvl.3-deficient mice (Kvl.3(-/-)) revealed a previously unrecognized role for Kv1.3 in body weight regulation. Kv1.3(-/-) mice weighed significantly less than control littermates (Xu et al., 2003). Moreover, knockout mice were protected from diet-induced obesity and gained significantly less weight than littermate controls when placed on a high-fat diet.

MacDaniel et al. (2001) reported an anorexic effect of K+ channel blockade by extracellular application of 4-aminopyridine (4-AP), a Kv-channel blocker, in mesenteric arterial smooth muscle (MASMC) and intestinal epithelial cells functionally expressing multiple Kv channel alpha- and beta-subunits including Kvbeta2.1 encoded by KCNAB2 in rats.

It has been demonstrated that the anorexic drugs, fenfluramine and dexenfluramine, in addition to inhibiting serotonin transporters (Baumann et al, 2000), decrease Kv channel activity in vascular smooth muscle cells (Hu et al, 1998, Michelakis et al, 1999; Wang et al., 1997). These observations suggest that the activity of Kv channels in MASMC may play an important role in the regulation of energy intake by controlling nutrient transportation.

Taken together, the linkage results provided in the present application, identifying the human KCNAB2 gene in the critical interval of genetic alterations linked to obesity on chromosome 1, with its involvement in the activity of voltage-gated potassium (Kv) channels, we conclude that alterations (e.g., mutations and/or polymorphisms) in the KCNAB2 gene or its regulatory sequences may contribute to the development of human obesity and represent a novel target for diagnosis or therapeutic intervention.

### 2. Association study

The same families that have been used for the linkage study were also used to test for association between a sepcific phenotype (here obesity) in question and the genetic marker allele or haplotypes containing a specific marker allele using the transmission disequilibrium test (TDT). The TDT is a powerful association test as it is insensitive to population stratification problems in the tested sample. Briefly, the segregation of alleles from heterozygous parents to their affected off-spring is tested. The portion of alleles transmitted to the affected offspring compared to the non-transmitted alleles is compared to the ratio expected under random distribution. A significant excess of allele transmission over the expected value is evidence for an association of the respective allele or haplotype with the studied obesity phenotype.

The results of this analysis show that certain alleles of the KCNAB2 gene are positively associated with obesity and might therefore increase the susceptibility to disease. In the tested population, the allele G of SNP33 and the allele A of SNP34 is correlated to obesity (LRS = 5.266, p = 0.02175 for SNP33 and LRS = 7.014, p = 0.008087 for SNP34, respectively) as determined by TDT. On the other hand, the allele A of SNP33 and the allele G of SNP34 is significantly under-transmitted to obese individuals indicating that this allele might help protecting from the disease
An example of the transmission of the alleles to obese individuals is given in table 4.

**Table 4**

| SNP | Allele | Frequency of allele transmitted to obese individuals | Frequency of allele not transmitted to obese individuals | Odds ratio | p- value |
|---|---|---|---|---|---|
| SNP33 | A | 0.5804 | 0.6784 | 1 | 0.02175 |
| SNP33 | G | 0.4196 | 0.3216 | 1.53 | 0.02175 |
| SNP34 | G | 0.5878 | 0.6985 | 1 | 0.008087 |
| SNP34 | A | 0.4122 | 0.3015 | 1.63 | 0.008087 |

In addition, haplotypes were constructed for SNP2, SNP17, SNP23, SNP34, SNP38, SNP42, SNP47, SNP49, SNP53 and SNP69 to identify the phase for all SNPs.

The results of this analysis showed that certain haplotypes, all characterized by the presence of allele A at SNP34 are strongly associated with obesity. In the tested population, haplotypes having allele A at SNP34 are strongly correlated to obesity (p=0.0004768 for G-A-A of SNP2-SNP34-SNP38, p=0.00588 for A-A-C of SNP34-SNP42-SNP51, p=0.003045 for A-G-G of SNP34- SNP47-SNP49 and p=0.005235 for G-C-C of SNP34-SNP53-SNP69, respectively, as determined by TDT), while certain haplotypes devoid of allele A are preferentially not transmitted to obese subjects (p=0.01393 for G-G-G of SNP2-SNP34-SNP38, p=0.01684 for C-G-A of SNP2-SNP34-SNP38, p=2.34e-06 for A-T-G of SNP17-SNP23-SNP34, p=0.03391 for G-G-C of SNP34-SNP42-SNP51, p=0.02026 for G-A-A of SNP34-SNP47-SNP49, and p=0.0008873 for A-G-T of SNP34-SNP53-SNP69, respectively). Haplotypes that carry allele G instead of allele A at SNP34 show significant evidence to be under-represented in obese subjects.

Examples of haplotypes with preferential transmission and non-transmission of SNP34 to obese individuals are given in table 5.

**Table 5**

| SNPs used to construct haplotype | Haplotype | Frequency of haplotype transmitted to obese individuals | Frequency of haplotype not transmitted to obese individuals | Odds Ratio | P-value |
|---|---|---|---|---|---|
| SNP2-**SNP34**-SNP38 | G-**G-**G | 0.1219 | 0.2171 | 1.0 | 0.01393 |
| SNP2-**SNP34**-SNP38 | G-**A**-A | 0.2284 | 0.1128 | 3.605 | 0.0004768 |
| SNP2-**SNP34**-SNP38 | C-**G-**A | 0.1019 | 0.1816 | 0.9993 | 0.01684 |
| SNP17-SNP23-**SNP34** | A-T-**G** | 0.1297 | 0.3137 | 0.3876 | 2.34e-06 |
| **SNP34**-SNP42-SNP51 | **G**-G-C | 0.03669 | 0.08217 | 0.5977 | 0.03391 |
| **SNP34**-SNP42-SNP51 | **A**-A-C | 0.2222 | 0.1195 | 2.489 | 0.00588 |
| **SNP34**-SNP47-SNP49 | **G**-A-A | 0.01247 | 0.0341 | 0.4484 | 0.02026 |
| **SNP34**-SNP47-SNP49 | **A**-G-G | 0.2869 | 0.1773 | 1.985 | 0.003045 |
| **SNP34**-SNP53-SNP69 | **G**-C-C | 0.1339 | 0.2508 | 0.8193 | 0.0008873 |
| **SNP34**-SNP53-SNP69 | **A**-G-T | 0.07859 | 0.02371 | 5.086 | 0.005235 |

### REFERENCES

Baumann MH, Ayestas MA, Dersch CM et al (2000) Serotonin transporters, serotonin release, and the mechanism of fenfluramine neurotoxicity. Ann N Y Acad Sci 914:172-86
Chagnon YC, Rankinen T, Snyder EE et al. (2003) The human obesity gene map: The 2002 update. Obes Res. 11(3):313-367.
Hebebrand J, Hescker H, Himmelmann GW, Schäfer H, Remschmidt H (1994) Percentiles for the body mass index based on data of the German national nutrition survey and a review of relevant factors with an influence on body weight. Aktuelle Ernahrungsmedizin 19: 259-265.
Hebebrand J, Himmelmann GW, Hescker H, Schäfer H, Remschmidt H (1996) Use of percentiles for the body mass index in anorexia nervosa : diagnostic, epidemiological and therapeutic considerations. Int J Eat Dis 19: 359-369.
Hebebrand J, Hinney A, Roth H et al. (1998) Genetische Aspekte der Adipositas. In:J Wechsler (ed) Adipositas. Ex Libris Roche. Blackwell Verlag: 105-118.
Hinney A, Schmidt A, Nottebom K, et al. (1999) Several mutations in the melanocortin-4 receptor gene including a nonsense and a frameshift mutation associated with dominantly inherited obesity in humans. J. Clin Endocrinol Metab. Apr; 84(4):1483-1486.
Hu S, Wang S, Gibson J et al (1998) Inhibition of delayed rectifier K+ channels by dexfenfluramine (Redux). J Pharmacol Exp Ther. 287(2):480-6.
Hugot JP, Chamaillard M, Zouali H et al. (2001) Association of NOD2 leucine-rich repeat variants with susceptibility to Crohn's disease. Nature 411(6837):599-603.
Huszar D, Lynch CA, Fairchild-Huntress V, et al. (1997) Targeted disruption of the melanocortin-4 receptor results in obesity in mice. Cell 88:131-141.
Lander E and Kruglyak L (1995) Genetic dissection of complex traits: guidelines for interpreting and reporting linkage results. Nat Genet, 11(3):241-247.
Lesage S, Zouali H, Cezard Jpet al. (2002) CARD15/NOD2 mutational analysis and genotype-phenotype correlation in 612 patients with inflammatory bowel disease. Am J Hum Genet. 70(4):845-857.
MacDonald PE and Wheeler MB (2003) Voltage-ependent K+ channels in pancreatic beta cells: Role, regulation and potential as therapeutic target. Diabetologia 46:1046-1062.
   Michelakis ED, Weir EK, Nelson DP et al (1999) Dexfenfluramine elevates systemic blood pressure by inhibiting potassium currents in vascular smooth muscle cells. J Pharmacol Exp Ther. 291(3):1143-9.
Rioux JD, Daly MJ, Silverberg MS et al. (2001) Genetic variation in the 5q31 cytokine gene cluster confers susceptibility to Crohn disease. Nat Genet 29(2): 223-228.
Rioux JD, Silverberg MS, Daly MJ (2000) Genomewide search in Canadian families with inflammatory bowel disease reveals two novel susceptibility loci. Am J Hum Genet 66(6):1863-1870.
Schneider R (1996) Relevanz und Kosten der Adipositas in Deutschland. Ernährungs-Umschau 43:369-374.
Vaisse C, Clément K, Guy-Grand B et al (1998) A frameshift mutation in human MC4R is associated with a dominant form of obesity. Nat Genet 20:113-114.
Wang J, Juhaszova M, Rubin LJ et al (1997) Hypoxia inhibits gene expression of voltage-gated K+ channel alpha subunits in pulmonary artery smooth muscle cells. J Clin Invest. 100(9):2347-53.
WHO (1998) Preventing and managing the global epidemic. WHO, Geneva.
Yeo GS, Farooqi IS, Aminian S, et al (1998) A frameshift mutation in MC4R associated with dominantly inherited human obesity. Nat Genet 20:111-112.
Wolf AM, Colditz GA (1996) Social and economic effects of body weight in the United States. Am J Clin Nutr 63(3 Suppl):4665-469S.
Xu J, Koni PA, Wang P et al (2003) The voltage-gated potassium channel Kv1.3 regulates energy homeostasis and body weight. Hum Mol Genet 12(5):551-9.
Zhang Y, Proenca R, Maffei M, Barone M, Leopold L, Friedman JM. (1994) Positional cloning of the mouse obese gene and its human homologue. Nature 372: 425-433.

## Claims

1. An *in vitro* method of detecting the presence of or predisposition to obesity in a subject, the method comprising detecting the presence of an alteration in the KCNAB2 gene locus in a sample from the subject.

2. The method of claim 1, wherein the presence of an alteration in the KCNAB2 gene locus is detected by sequencing, selective hybridisation and/or selective amplification.

3. The method of any one of claims 1-2, wherein said alteration is one or several SNP(s) or an haplotype of SNPs associated with obesity.

4. The method of claim 3, wherein said haplotype associated with obesity comprises several SNPs selected in the group consisting of SNP2, SNP17, SNP23, SNP33, SNP34, SNP38, SNP42, SNP47, SNP49, SNP51, SNP53 and SNP69.

5. The method of claim 3, wherein said SNP associated with obesity is SNP33 or SNP34.

6. The method of claim 5, wherein said SNP associated with obesity is allele G of SNP33.

7. The method of claim 5, wherein said SNP associated with obesity is allele A of SNP34.

## Patentansprüche

1. *In vitro* Verfahren zur Detektion der Anwesenheit von oder Prädisposition für Adipositas in einem Subjekt, wobei das Verfahren den Schritt umfasst, die Anwesenheit einer Veränderung im KCNAB2 Genlokus in einer Probe des Subjekts zu detektieren.

2. Das Verfahren nach Anspruch 1, wobei die Anwesenheit einer Veränderung im KCNAB2 Genlokus durch Sequenzierung, selektive Hybridisierung und/oder selektive Amplifikation detektiert wird.

3. Das Verfahren nach einem der Ansprüche 1-2, wobei die Veränderung, die mit Adipositas assoziiert ist, ein oder mehrere SNP(s) oder ein Haplotyp von SNPs ist.

4. Das Verfahren nach Anspruch 3, wobei der Haplotyp, der mit Adipositas assoziiert ist, mehrere SNPs umfasst, wobei die SNPs ausgewählt sind aus SNP2, SNP17, SNP23, SNP33, SNP34, SNP38, SNP42, SNP47, SNP49, SNP51, SNP53 und SNP69.

5. Das Verfahren nach Anspruch 3, wobei der SNP, der mit Adipositas assoziiert ist, SNP33 oder SNP34 ist.

6. Das Verfahren nach Anspruch 5, wobei der SNP, der mit Adipositas assoziiert ist, Allel G von SNP33 ist.

7. Das Verfahren nach Anspruch 5, wobei der SNP, der mit Adipositas assoziiert ist, Allel A von SNP34 ist.

## Revendications

1. Méthode *in vitro* de détection de la présence ou d'une prédisposition à l'obésité chez un sujet, la méthode comprenant la détection de la présence d'une altération dans le locus du gène KCNAB2 dans un échantillon du sujet.

2. Méthode selon la revendication 1, dans laquelle la présence d'une altération dans le locus du gène KCNAB2 est détectée par séquençage, hybridation sélective et/ou amplification sélective.

3. Méthode selon l'une quelconque des revendications 1-2, dans laquelle ladite altération est un ou plusieurs SNP(s) ou un haplotype of SNPs associé à l'obésité.

4. Méthode selon la revendication 3, dans laquelle ledit haplotype associé à l'obésité comprend plusieurs SNPs choisis parmi le groupe consistant en SNP2, SNP17, SNP23, SNP33, SNP34, SNP38, SNP42, SNP47, SNP49, SNP51, SNP53 and SNP69.

5. Méthode selon la revendication 3, dans laquelle ledit SNP associé à l'obésité est SNP33 ou SNP34.

6. Méthode selon la revendication 5, dans laquelle ledit SNP associé à l'obésité est l'allèle G de SNP33.

7. Méthode selon la revendication 5, dans laquelle ledit SNP associé à l'obésité est l'allèle A de SNP34.
